# EUROPEAN PATENT APPLICATION

(11) **EP 2 085 070 A1**
(43) Date of publication of application: **05.08.2009**
(21) Application number: 08150189.2
(22) Date of filing: 11.01.2008
(51) Int. Cl.: A61K 8/66, A61Q 19/10, C11D 3/386

(54) **Cleaning and/or treatment compositions**

(71) Applicant: Procter & Gamble International Operations SA., 1213 Petit-Lancy 1 (CH)
(72) Inventor: Souter, Philip Frank, Newcastle upon Tyne, NE65 8UP (GB); Perez-Prat Vinuesa, Eva Maria, 1180, Brussels (BE); Herrero Acero, Enrique, 50017, Zaragoza (ES); Pricelius, Sina, 8010, Graz (AT); Guebitz, Georg Maximilian, 8047, Graz (AT)
(74) Representative: Howard, Phillip Jan

(57) **Abstract**

This invention relates to compositions comprising certain cellobiose dehydrogenase enzymes and processes for making and using such compositions, including the use of such compositions to clean and/or treat a situs.

## Description

### FIELD OF INVENTION

This invention relates to compositions comprising cellobiose dehydrogenase enzymes and processes for making and using such compositions.

### BACKGROUND OF THE INVENTION

Enzymes can be used to improve the cleaning properties of compositions. Unfortunately, while there is a need for improved bleaching, a cleaning composition comprising an enzyme that provides effective and efficient bleaching has yet to be delivered.

Surprisingly, when a cellobiose dehydrogenase enzyme is incorporated, in accordance with the teaching of the present specification, in a cleaning composition, such composition delivers effective and efficient bleaching.

### SUMMARY OF THE INVENTION

This invention relates to compositions comprising certain cellobiose dehydrogenase enzymes and processes for making and using such compositions. When compositions that comprise certain cellobiose dehydrogenase enzymes are formulated in accordance with the teachings of the present invention, such compositions can provide effective and efficient bleaching.

### DETAILED DESCRIPTION OF THE INVENTION

### Definitions

As used herein, the term "cleaning composition" includes, unless otherwise indicated, granular or powder-form all-purpose or "heavy-duty" washing agents, especially laundry detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, laundry bars, mouthwashes, denture cleaners, car or carpet shampoos, bathroom cleaners; hair shampoos and hair-rinses; shower gels and foam baths and metal cleaners; as well as cleaning auxiliaries such as bleach additives, laundry additives or pre-treats and "stain-stick" or pre-treat types.

As used herein, the phrase "is independently selected from the group consisting of ....." means that moieties or elements that are selected from the referenced Markush group can be the same, can be different or any mixture of elements.

As used herein, the term "cellobiose dehydrogenase enzymes" or "CDH" refers to enzymes that are capable of oxidizing one or more of the below substrates:
a) oligosaccharides selected from the group consisting of cello-oligosaccharides, including a cello-oligosaccharide selected from the group consisting of cellobiose, cello-triose, cello-tetraose, cello-pentaose, cello-hexaose, cellodextrin and mixtures thereof, lactose, maltose, xylobiose and mixtures thereof;
b) amorphous cellulose;
c) cellulosic polymers including carboxy methyl cellulose; and
d) mixtures thereof, and having an EC. classification of EC. 1.1.99.18 or EC 1.1.5.1, the latter being a proteolytic product of EC 1.1.99.18, and may be described as cellobiose dehydrogenase, cellobiose oxidase, cellobiose dehydrogenase (quinone), cellobiose oxidoreductase, or cellobiose-quinone oxidoreductase.

As used herein, the term "cellobiohydrolase domain" refers to the catalytic domain of enzymes, having an EC classification of EC 3.2.1.91, or EC 3.2.1.150, and that are capable of liberating cellobiose from different polysaccharide substrates including cellulose or xyloglucans.

The expression "carbohydrate-binding-module" or "CBM" refers to a module with affinity to carbohydrate that targets its associated enzyme to the carbohydrate.

As used herein, the articles "a" and "an" when used in a claim, are understood to mean one or more of what is claimed or described.

As used herein, the terms "include", "includes" and "including" are meant to be synonymous with the phrase "including but no limited to".

Unless otherwise noted, all component or composition levels are in reference to the active level of that component or composition, and are exclusive of impurities, for example, residual solvents or by-products, which may be present in commercially available sources.

Unless otherwise noted, the cellobiose dehydrogenase enzymes of the present invention are expressed in terms of active protein level and are exclusive of impurities, for example, residual solvents or by-products, which may be present in commercially available sources.

All percentages and ratios are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

### Compositions

In one aspect, a composition that may comprise one or more adjunct ingredients and a cellobiose dehydrogenase enzyme said composition comprising, based on total composition weight, 0% weight % cellulase or said composition having a mass ratio of cellobiose dehydrogenase enzyme:cellulase of from about 0.01 to about 1,000,000, from about 0.02 to about 500,000, from about 0.05 to about 200,000 or even from about 0.1 to about 10,000 is disclosed.

In one aspect of such composition, said cellobiose dehydrogenase may have a minimum of 60% amino acid sequence identity with the wild-type cellobiose dehydrogenases derived from a microorganism selected from the group consisting of *Myceliophtore thermophila, Phanerochaete chrysosporium, Trametes versicolor, Grifola frondosa, Neosartorya fischeri, Humicola insolens, Sclerotium rolfsii, Myriococcum thermophilum* and combinations thereof.

In one aspect of such composition said cellobiose dehydrogenase may have a minimum of 70% amino acid sequence identity with any enzyme having a sequence corresponding to a sequence ID selected from the group consisting of SEQ. ID Nos. 1, 2, 3, 4, 5, 6, or 7 .

In one aspect of such composition said cellobiose dehydrogenase enzyme may have a minimum of 85% amino acid sequence identity with a sequence corresponding to a sequence ID selected from the group consisting of SEQ. ID Nos. 1,2, or amino acids 22 to 785 of SEQ. ID No. 3.

In one aspect of such composition, said composition may comprise a material selected from the group consisting of :
a) oligosaccharides selected from the group consisting of cello-oligosaccharides, including a cello-oligosaccharides selected from the group consisting of cellobiose, cello-triose, cello-tetraose, cello-pentaose, cello-hexaose, cellodextrin and mixtures thereof, lactose, maltose, xylobiose and mixtures thereof;
b) amorphous cellulose;
c) cellulosic polymers including carboxy methyl cellulose; and
d) mixtures thereof.

In one aspect of such composition, said cellobiose dehydrogenase enzyme may comprise a carbohydrate binding module, such as a cellulose-binding domain.

In one aspect of such composition said cellobiose dehydrogenase may comprise a cellobiose dehydrogenase domain and a cellobiohydrolase domain.

In one aspect of such composition said one or more adjunct ingredients may be selected from the group consisting of surfactants, builders, chelating agents, dye transfer inhibiting agents, dispersants, additional enzymes, and enzyme stabilizers, catalytic materials, bleaching agents, polymeric dispersing agents, clay soil removal/anti-redeposition agents, brighteners, suds suppressors, dyes, perfumes, structure elasticizing agents, fabric softeners, carriers, hydrotropes, processing aids, solvents, pigments, hueing agents, photobleaches, structurants, and mixtures thereof.

In one aspect of such composition, said one or more adjunct ingredients may comprise an enzyme. In one aspect of such composition said enzyme may be selected from the group consisting of peroxidases, proteases, lipases, phospholipases, cellobiohydrolases, esterases, cutinases, pectinases, mannanases, pectate lyases, keratinases, reductases, oxidases, phenoloxidases, lipoxygenases, ligninases, pullulanases, tannases, pentosanases, glucanases, arabinosidases, hyaluronidase, chondroitinase, laccases, amylases, or mixtures thereof. In one aspect of such composition, said enzyme may be selected from the group consisting of:
a.) lipases, such as lipases having greater than 90% amino acid sequence identity to SEQ. ID 8;
b.) alpha-amylases, such as alpha-amylases having greater than 90% amino acid sequence identity to SEQ. ID 9;
c.) endoglucanases, such as endoglucanases having greater than 90% amino acid sequence identity to SEQ. ID 10;
d.) proteases, including metalloproteases, neutral or alkaline serine proteases, such as subtilisins including those derived from *Bacillus lentus, B. alkalophilus, B. subtilis, B. amyloliquefaciens*, and mixtures thereof; and
e.) mixtures thereof.

In one aspect of such composition, said one or more adjunct ingredients may comprise a surfactant, such as a surfactant being selected from the group of:
a.) anionic surfactants including anionic surfactants selected from the group consisting of linear alkylbenzene-sulfonate (LAS), alcohol ethoxysulfate (AES), mid-branched alkyl sulfates (HSAS) and mixtures thereof;
b.) non ionic surfactants including alcohol ethoxylates, said alcohol ethoxylates, in one aspect, having a chain length of from 1 to 18 carbons;
c.) amine oxides and mixtures thereof.

In one aspect of such composition, said composition may comprise a polymer. In one aspect, said polymer may be selected from the group consisting of
a.) polyacrylates;
b.) maleic/acrylic acid copolymers;
c.) cellulose-derived polymers, including cellulose-derived polymers selected from the group consisting of carboxymethylcellulose, methyl hydroxyethylcellulose and mixtures thereof;
d.) polyethyleneimine polymers;
e.) soil release polymers, including co-polymers comprising two or more monomers selected from the group consisting of sulfo-isophthalate, ethylene terephthalate, oxyethyleneterephthalate and mixtures thereof; and
f.) mixtures thereof.

In one aspect of such composition, said composition may comprise a builder. In one aspect, said builder may be selected from the group consisting of
a.) citric acid;
b.) C₁₂-C₁₈ fatty acids;
c.) aluminosilicates, in one aspect a zeolite selected from the group consisting of Zeolite A, Zeolite X , Zeolite Y and mixtures thereof;
d.) sodium tripolyphosphate; and
e.) mixtures thereof.

In one aspect of such composition, said composition may comprise, based on total product weight, less than about 15%, less than about 10%, less than about 8% or even from about 0.01 % to about 7% builder.

In one aspect of such composition, said composition may comprise a material selected from the group consisting of a photobleach, a fabric hueing agent and mixtures thereof. In one aspect said photobleach may be being selected from the group consisting of
a.) xanthene dyes and mixtures thereof;
b.) sulfonated zinc phthalocyanine, sulfonated aluminium phthalocyanine, Eosin Y, Phoxine B, Rose Bengal, C.I. Food Red 14 and mixtures thereof;
c.) water soluble phthalocyanine; and
d.) mixtures thereof; and said fabric hueing agent may be selected from the group consisting of

a.) dyes, in one aspect said dyes being selected from the group consisting of small molecule dyes, polymeric dyes and mixtures thereof;
b.) dye-clay conjugates comprising at least one cationic/basic dye and a smectite; clay; and
c.) mixtures thereof; and mixtures thereof.

In one aspect, said composition may comprise a bleaching agent selected from one or more of the following:
a) a source of hydrogen peroxide;
b) a bleach activator, including tetraacetyl ethylene diamine (TAED) and /or nonanoyloxybenzene sulphonate (NOBS);
c) a pre-formed peracid;
d) a diacyl peroxide;
e) a tetraacyl peroxide; and
f) mixtures thereof.

In one aspect of such composition, said composition may comprise a bleach catalyst that is capable of accepting an oxygen atom from a peroxyacid and transferring the oxygen atom to an oxidizeable substrate.

In one aspect of the aforementioned compositions, any enzyme component may be encapsulated and/or provided in prill form.

In one aspect of the aforementioned compositions, such compositions may take any form, for example, a solid including a granule or powder or a fluid including a liquid, paste or gel.

In one aspect of the aforementioned compositions, such compositions may be cleaning composition and/or treatment compositions.

The compositions disclosed herein may have any acceptable combination of parameters described above in this section of the present specification entitled "Compositions"

Suitable cellobiose dehydrogenase enzymes for use in the compositions of the present specification include the cellobiose dehydrogenase enzymes described in the section of the present specification entitled "Suitable cellobiose dehydrogenase enzymes"

Without wishing to be bound by theory, compositions formulated in accordance with the teachings of the present specification are believed to be particularly effective at stain removal of bleachable soils such as fruit (*e*.*g*. blueberry), red wine, tea or coffee. Such compositions are believed to be particularly effective at providing whiteness and/or dingy cleaning benefits. Furthermore, it is believed that certain cellobiose dehydrogenase enzymes are unexpectedly stable in cleaning compositions and thus can provide cleaning compositions with such superior cleaning properties. Without wishing to be bound by theory, such cellobiose dehydrogenase enzymes are believed to be able to generate oxidizing species at the cotton fabric and/or soil surface and thereby impart superior cleaning than if the equivalent bleaching species are added in solution.

### Suitable cellobiose dehydrogenase enzymes

Suitable cellobiose dehydrogenase enzymes can be isolated from one of *Myceliophtore thermophila, Phanerochaete chrysosporium, Trametes versicolor, Grifola frondosa, Aspergillus Niger, Neosartorya fischeri, Humicola insolens, Sclerotium rolfsii* and *Myriococcum thermophilum*. Examples of such enzymes are described in the literature in J. Biotechnology 78 (2000) 93-113, Applied and Environmental Microbiology, Apr. 2001, p.1766-1774, USP 6,033,981 and in Electroanalysis 19, 2 - 3, 2007, 172 - 180.

Suitable cellobiose dehydrogenase enzymes include haemoflavoproteins that in one aspect belong to EC Class 1.1.99.18 and are active under typical wash conditions.

Suitable cellobiose dehydrogenase enzymes may be protein engineered to improve their stability in detergents by techniques well known in the art including the ones described in USP 5,324,653 and USP 7,163,816 B2.

Suitable cellobiose dehydrogenase enzymes may have greater than 60%, or greater than 70% or greater than 80%, or greater than 90% or greater than 95% or greater than 98% amino acid sequence identity with the enzymes whose sequences are shown as SEQ. ID 1 - 7. For the purpose of the present invention the degree of identity between amino acid sequences is determined using the BLASTP algorithm (Basic Local Alignment Search Tool) and/or the BLAST 2 Sequences tool described in Tatusova & Madden (1999) FEMS Microb. Lett. 174(2):247-250 both freely available on the NCBI website whose web address is shown in parentheses (http://www.ncbi.nlm.nih.gov/BLAST/). The parameters used for sequence alignment are the default settings in the mentioned web site.

Suitable cellobiose dehydrogenases may contain a CBM such as a cellulose-binding domain.

Suitable cellobiose dehydrogenase enzymes may also be fused to a further catalytic domain having cellulolytic, hemicellulolytic, or cellobiohydrolase activity, optionally via a linker molecule.

The aforementioned enzymes can be provided either in the form of a low-dusting solid (typically a granule or prill) or as a stabilized liquid or as a protected liquid or encapsulated enzyme. Numerous techniques are described in the art to produce low-dusting solid forms of enzymes, including prilling, extrusion, spheronization, drum granulation and fluid bed spray coating and exemplified in USP 4,106,991; USP 4,242,219; USP 4,689,297, USP 5,324,649 and USP 7,018,821 B2. Liquid enzyme preparations may, for instance, be stabilized by adding a polyol such as propylene glycol, a sugar or sugar alcohol, lactic acid or boric acid according to established methods. Protected liquid enzymes or encapsulated enzymes may be prepared according to the methods disclosed in USP 4,906,396, USP 6,221,829 B1, USP 6,359,031 B1 and USP 6,242,405 B1.

### Adjunct Materials

While not essential for the purposes of the present invention, the non-limiting list of adjuncts illustrated hereinafter are suitable for use in the instant compositions and may be desirably incorporated in certain embodiments of the invention, for example to assist or enhance cleaning performance, for treatment of the substrate to be cleaned, or to modify the aesthetics of the cleaning composition as is the case with perfumes, colorants, dyes or the like. The precise nature of these additional components, and levels of incorporation thereof, will depend on the physical form of the composition and the nature of the cleaning operation for which it is to be used. Suitable adjunct materials include, but are not limited to, surfactants, builders, chelating agents, dye transfer inhibiting agents, dispersants, additional enzymes, and enzyme stabilizers, catalytic materials, bleach activators, hydrogen peroxide, sources of hydrogen peroxide, preformed peracids, polymeric dispersing agents, clay soil removal/anti-redeposition agents, brighteners, suds suppressors, dyes, photobleaches, perfumes, structure elasticizing agents, fabric softeners, carriers, hydrotropes, processing aids, solvents, hueing agents, structurants and/or pigments. In addition to the disclosure below, suitable examples of such other adjuncts and levels of use are found in U.S. Patent Nos. 5,576,282, 6,306,812 B1 and 6,326,348 B1 that are incorporated by reference.

As stated, the adjunct ingredients are not essential to Applicants' compositions. Thus, certain embodiments of Applicants' compositions do not contain one or more of the following adjuncts materials: surfactants, builders, chelating agents, dye transfer inhibiting agents, dispersants, additional enzymes, and enzyme stabilizers, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids, solvents and/or pigments. However, when one or more adjuncts are present, such one or more adjuncts may be present as detailed below:
Bleaching Agents and Non-metal Bleach Catalysts- The cleaning compositions of the present invention may comprise one or more bleaching agents. Suitable bleaching agents other than bleaching catalysts include photobleaches, bleach activators, hydrogen peroxide, sources of hydrogen peroxide, pre-formed peracids and mixtures thereof. In general, when a bleaching agent is used, the compositions of the present invention may comprise from about 0.1% to about 50% or even from about 0.1% to about 25% bleaching agent by weight of the subject cleaning composition. Examples of suitable bleaching agents include:
   (1) preformed peracids: Suitable preformed peracids include, but are not limited to, compounds selected from the group consisting of percarboxylic acids and salts, percarbonic acids and salts, perimidic acids and salts, peroxymonosulfuric acids and salts, for example, Oxone ®, and mixtures thereof. Suitable percarboxylic acids include hydrophobic and hydrophilic peracids having the formula R-(C=O)O-O-M wherein R is an alkyl group, optionally branched, having, when the peracid is hydrophobic, from 6 to 14 carbon atoms, or from 8 to 12 carbon atoms and, when the peracid is hydrophilic, less than 6 carbon atoms or even less than 4 carbon atoms; and M is a counterion, for example, sodium, potassium or hydrogen;
   (2) sources of hydrogen peroxide, for example, inorganic perhydrate salts, including alkali metal salts such as sodium salts of perborate (usually mono- or tetra-hydrate), percarbonate, persulphate, perphosphate, persilicate salts and mixtures thereof. In one aspect of the invention the inorganic perhydrate salts are selected from the group consisting of sodium salts of perborate, percarbonate and mixtures thereof. When employed, inorganic perhydrate salts are typically present in amounts of from 0.05 to 40 wt%, or 1 to 30 wt% of the overall composition and are typically incorporated into such compositions as a crystalline solid that may be coated. Suitable coatings include, inorganic salts such as alkali metal silicate, carbonate or borate salts or mixtures thereof, or organic materials such as water-soluble or dispersible polymers, waxes, oils or fatty soaps; and
   (3) bleach activators having R-(C=O)-L wherein R is an alkyl group, optionally branched, having, when the bleach activator is hydrophobic, from 6 to 14 carbon atoms, or from 8 to 12 carbon atoms and, when the bleach activator is hydrophilic, less than 6 carbon atoms or even less than 4 carbon atoms; and L is leaving group. Examples of suitable leaving groups are benzoic acid and derivatives thereof - especially benzene sulphonate. Suitable bleach activators include dodecanoyl oxybenzene sulphonate, decanoyl oxybenzene sulphonate, decanoyl oxybenzoic acid or salts thereof, 3,5,5-trimethyl hexanoyloxybenzene sulphonate, tetraacetyl ethylene diamine (TAED) and nonanoyloxybenzene sulphonate (NOBS). Suitable bleach activators are also disclosed in WO 98/17767. While any suitable bleach activator may be employed, in one aspect of the invention the subject cleaning composition may comprise NOBS, TAED or mixtures thereof.
   (4) Suitable non-metal bleach catalysts and appropriate levels of such catalysts for use in the present compositions are disclosed in USP 7,169,744 B2 and USPA 2006/0287210 A1.

When present, the peracid and/or bleach activator is generally present in the composition in an amount of from about 0.1 to about 60 wt%, from about 0.5 to about 40 wt % or even from about 0.6 to about 10 wt% based on the composition. One or more hydrophobic peracids or precursors thereof may be used in combination with one or more hydrophilic peracid or precursor thereof.

The amounts of hydrogen peroxide source and peracid or bleach activator may be selected such that the molar ratio of available oxygen (from the peroxide source) to peracid is from 1:1 to 35:1, or even 2:1 to 10:1.

Surfactants - The cleaning compositions according to the present invention may comprise a surfactant or surfactant system wherein the surfactant can be selected from nonionic surfactants, anionic surfactants, cationic surfactants, ampholytic surfactants, zwitterionic surfactants, semi-polar nonionic surfactants and mixtures thereof. When present, surfactant is typically present at a level of from about 0.1% to about 60%, from about 1% to about 50% or even from about 5% to about 40% by weight of the subject composition.

Builders - The cleaning compositions of the present invention may comprise one or more detergent builders or builder systems. When a builder is used, the subject composition will typically comprise at least about 1%, from about 5% to about 60% or even from about 10% to about 40% builder by weight of the subject composition.

Builders include, but are not limited to, the alkali metal, ammonium and alkanolammonium salts of polyphosphates, alkali metal silicates, alkaline earth and alkali metal carbonates, aluminosilicate builders and polycarboxylate compounds, ether hydroxypolycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, citric acid, oxydisuccinic acid, polymaleic acid, benzene 1,3,5-tricarboxylic acid, carboxymethyloxysuccinic acid, and soluble salts thereof.

Chelating Agents - The cleaning compositions herein may contain a chelating agent. Suitable chelating agents include copper, iron and/or manganese chelating agents and mixtures thereof. When a chelating agent is used, the subject composition may comprise from about 0.005% to about 15% or even from about 3.0% to about 10% chelating agent by weight of the subject composition.

Dye Transfer Inhibiting Agents - The cleaning compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in a subject composition, the dye transfer inhibiting agents may be present at levels from about 0.0001 % to about 10%, from about 0.01 % to about 5% or even from about 0.1% to about 3% by weight of the composition.

Brighteners - The cleaning compositions of the present invention can also contain additional components that may tint articles being cleaned, such as fluorescent brighteners. Suitable fluorescent brightener levels include lower levels of from about 0.01, from about 0.05, from about 0.1 or even from about 0.2 wt % to upper levels of 0.5 or even 0.75 wt %.

Dispersants - The compositions of the present invention can also contain dispersants. Suitable water-soluble organic materials include the homo- or co-polymeric acids or their salts, in which the polycarboxylic acid comprises at least two carboxyl radicals separated from each other by not more than two carbon atoms.

Polymers - The compositions of the present invention can also contain one or more polymers. These would include soil release polymers & soil suspension polymers. This would include polymers commonly known in the art such as block polyesters according to USP 4,702,857 and sulfonated linear terephthalate ester oligomers according to USP 4,968,451, as well as ethoxylated tetraethylene pentaimine (EO₁₅₋₁₈) according to USP 4,597,898 and ethoxylated hexamethylene diamine available under the tradename LUTENSIT® from BASF and such as those described in WO 01/05874.

In one aspect, the soil release polymers may be co-polymers comprising two or more monomers selected from sulfo-isophthalate, ethylene terephthalate and oxyethyleneterephthalate, including those sold by Clariant under the names TexCare® SRA and TexCare® SRN tradenames, such as TexCare® SRA300 and TexCare® SRN100.

In a further aspect the polymers would include Poly ethtlene glycol/ alkyl acetate copolymers, including PEGVAc co-polymers such as those sold by BASF under the Sokalan tradename, e.g. Sokalan HP22.

In a further aspect the polymers would include cellulosic-based polymers such as amorphous cellulose and anionically and/or nonionically and/or cationically modified celluloses. Non-limiting examples of anionically suitable modified cellulose derivatives are the sodium or potassium salts of carboxymethyl cellulose, carboxyethyl cellulose, sulfoethyl cellulose, sulfopropyl cellulose, cellulose sulfate, phosphorylated cellulose, carboxymethyl hydroxyethyl cellulose, carboxymethyl hydroxypropyl cellulose, sulfoethyl hydroxyethyl cellulose, sulfoethyl hydroxypropyl cellulose, carboxymethyl methyl hydroxyethyl cellulose, carboxymethyl methyl cellulose, sulfoethyl methyl hydroxyethyl cellulose, sulfoethyl methyl cellulose, carboxymethyl ethyl hydroxyethyl cellulose, carboxymethyl ethyl cellulose, sulfoethyl ethyl hydroxyethyl cellulose, sulfoethyl ethyl cellulose, carboxymethyl methyl hydroxypropyl cellulose, sulfoethyl methyl hydroxypropyl cellulose, carboxymethyl dodecyl cellulose, carboxymethyl dodecoyl cellulose, carboxymethyl cyanoethyl cellulose and sulfoethyl cyanoethyl cellulose, Non-limiting examples of nonionically suitable modified cellulose derivatives include methyl cellulose, ethyl cellulose, propyl cellulose, hydroxyethyl cellulose, hydroxypropyl cellulose, methyl hydroxyethyl cellulose, ethyl hydroxyethyl cellulose, dodecyl hydroxyethyl cellulose, ethyl hydroxypropyl cellulose, cellulose acetate, methyl hydroxypropyl cellulose, methyl ethyl hydroxyethyl cellulose, butyl glycidyl ether-hydroxyethyl cellulose, and lauryl glycidyl ether-hydroxyethyl cellulose

Specific examples include Finnfix BDA (from Noviant), Dencel DT (from Denkim, Denizli, Turkey), Tylose CR1500 G2 (from Clariant), Carbose codes D65, D72, LT-30 and LT-20 (from Penn Carbose); hydrophobically modified cellulose derivatives for example as described in USP 6,600,033 B1; cellulose derivatives modified with polyethylene glycol, for example as described in DE102004063766.

Enzymes - The cleaning compositions can comprise one or more enzymes which provide cleaning performance and/or fabric care benefits. Examples of suitable enzymes include, but are not limited to, hemicellulases, peroxidases, proteases, xylanases, lipases, phospholipases, esterases, cutinases, pectinases, mannanases, pectate lyases, keratinases, reductases, oxidases, phenoloxidases, lipoxygenases, ligninases, pullulanases, tannases, pentosanases, malanases, β-glucanases, arabinosidases, hyaluronidase, chondroitinase, laccase, and amylases, or mixtures thereof. The enzyme may also be a cellulase, wherein the ratio by mass of cellobiose dehydrogenase: cellulase is between 0.05 and 1000000. A typical combination is an enzyme cocktail that may comprise, for example, a protease and lipase in conjunction with amylase. When present in a cleaning composition, the aforementioned additional enzymes may be present at levels from about 0.00001% to about 2%, from about 0.0001% to about 1% or even from about 0.001% to about 0.5% enzyme protein by weight of the composition.

Photobleaches - The cleaning compositions can comprise one or more photobleaches which provide cleaning performance. Examples of suitable catalytic photobleaches are disclosed in USPA 2007/0191249 A1, which is incorporated herein by reference, and include xanthene dyes, sulfonated zinc phthalocyanine, sulfonated aluminium phthalocyanine, Eosin Y, Phoxine B, Rose Bengal, C.I. Food Red 14, photo-initiators selected from the group consisting of Aromatic 1,4-quinones such as anthraquinones and naphthaquinones; Alpha amino ketones, particularly those containing a benzoyl moiety, otherwise called alpha-amino acetophenones; Alpha-hydroxy ketones, particularly alpha-hydroxy acetophenones; Phosphorus-containing photoinitiators, including monoacyl, bisacyl and trisacyl phosphine oxide and sulphides; Dialkoxy acetophenones; Alpha-haloacetophenones; Trisacyl phosphine oxides; Benzoin and benzoin based photoinitiators, 2-ethyl anthraquinone; Vitamin K3; 2-sulphate-anthraquinone; 2-methyl1-[4-phenyl]-2-morpholinopropan-1-one (Irgacure® 907); (2-benzyl-2-dimethyl amino-1-(4-morpholinophenyl)-butan-1-one (Irgacure® 369); (1-[4-(2-hydroxyethoxy)-phenyl]-2 hydroxy-2-methyl-1-propan-1-one) (Irgacure® 2959); 1-hydroxy-cyclohexyl-phenyl-ketone (Irgacure® 184); oligo[2-hydroxy 2-methyl-1-[4(1-methyl)-phenyl] propanone (Esacure® KIP 150); 2-4-6-(trimethylbenzoyl)diphenyl- phosphine oxide, bis(2,4,6-trimethylbenzoyl)-phenyl-phosphine oxide (Irgacure® 819); (2,4,6 trimethylbenzoyl)phenyl phosphinic acid ethyl ester (Lucirin® TPO-L); and mixtures thereof.

The aforementioned photobleaches can be used in combination (any mixture of photobleaches can be used). Suitable photobleaches can be purchased from Aldrich, Milwaukee, Wisconsin, USA; Frontier Scientific, Logan, Utah, USA; Ciba Specialty Chemicals, Basel, Switzerland; BASF, Ludwigshafen, Germany; Lamberti S.p.A, Gallarate, Italy; Dayglo Color Corporation, Mumbai, India; Organic Dyestuffs Corp., East Providence, Rhode Island, USA; and/or made in accordance with the examples contained herein.

Hueing dyes - The cleaning compositions can comprise one or more hueing dyes which can alter the tint of a surface as they absorb at least a portion of the visible light spectrum and thereby provide a consumer-preferred whiteness benefit. Examples of such hueing dyes are disclosed in USPA 2007/0129150 A1, which is incorporated herein by reference, and include dyes and dye-clay conjugates, and may also include pigments. Suitable hueing dyes include:
(a) Small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, such as Direct Violet Colour Index (Society of Dyers and Colourists, Bradford, UK) numbers Direct Violet 9, Direct Violet 35, Direct Violet 48, Direct Violet 51, Direct Violet 66, Direct Blue 1, Direct Blue 71, Direct Blue 80, Direct Blue 279, Acid Red 17, Acid Red 73, Acid Red 88, Acid Red 150, Acid Violet 15, Acid Violet 17, Acid Violet 24, Acid Violet 43, Acid Violet 49, Acid Blue 15, Acid Blue 17, Acid Blue 25, Acid Blue 29, Acid Blue 40, Acid Blue 45, Acid Blue 75, Acid Blue 80, Acid Blue 83, Acid Blue 90 and Acid Blue 113, Acid Black 1, Basic Violet 1, Basic Violet 3, Basic Violet 4, Basic Violet 10, Basic Violet 35, Basic Blue 3, Basic Blue 16, Basic Blue 22, Basic Blue 47, Basic Blue 66, Basic Blue 75, Basic Blue 159, Acid Violet 17, Acid Violet 43, Acid Red 73, Acid Red 88, Acid Red 150, Acid Blue 25, Acid Blue 29, Acid Blue 45, Acid Blue 113, Acid Black 1, Direct Blue 1, Direct Blue 71 and Direct Violet 51.
(b) Polymeric dyes include polymeric dyes selected from the group consisting of polymers containing conjugated chromogens (dye-polymer conjugates) and polymers with chromogens co-polymerised into the backbone of the polymer and mixtures thereof such as fabric-substantive colorants sold under the name of Liquitint® (Milliken, Spartanburg, South Carolina, USA), dye-polymer conjugates formed from at least one reactive dye and a polymer selected from the group consisting of polymers comprising a moiety selected from the group consisting of a hydroxyl moiety, a primary amine moiety, a secondary amine moiety, a thiol moiety and mixtures thereof. In still another aspect, suitable polymeric dyes include polymeric dyes selected from the group consisting of Liquitint® (Milliken, Spartanburg, South Carolina, USA) Violet CT, carboxymethyl cellulose (CMC) conjugated with a reactive blue, reactive violet or reactive red dye such as CMC conjugated with C.I. Reactive Blue 19, sold by Megazyme, Wicklow, Ireland under the product name AZO-CM-CELLULOSE, product code S-ACMC and mixtures thereof.
(c) Dye clay conjugates include dye clay conjugates selected from the group comprising at least one cationic/basic dye and a smectite clay, and mixtures thereof.
(d) Pigments such as Ultramarine Blue (C.I. Pigment Blue 29), Ultramarine Violet (C.I. Pigment Violet 15) and mixtures thereof.

Suitable fabric hueing agents can be purchased from Aldrich, Milwaukee, Wisconsin, USA; Ciba Specialty Chemicals, Basel, Switzerland; BASF, Ludwigshafen, Germany; Dayglo Color Corporation, Mumbai, India; Organic Dyestuffs Corp., East Providence, Rhode Island, USA; Dystar, Frankfurt, Germany; Lanxess, Leverkusen, Germany; Megazyme, Wicklow, Ireland; Clariant, Muttenz, Switzerland; Milliken, Spartanburg, South Carolina, USA and Avecia, Manchester, UK.

Enzyme Stabilizers - Enzymes for use in detergents can be stabilized by various techniques. The enzymes employed herein can be stabilized by the presence of water-soluble sources of calcium and/or magnesium ions in the finished compositions that provide such ions to the enzymes. In case of aqueous compositions comprising protease, a reversible protease inhibitor, such as a boron compound, can be added to further improve stability.

Catalytic Metal Complexes - Applicants' cleaning compositions may include catalytic metal complexes. One type of metal-containing bleach catalyst is a catalyst system comprising a transition metal cation of defined bleach catalytic activity, such as copper, iron, titanium, ruthenium, tungsten, molybdenum, or manganese cations, an auxiliary metal cation having little or no bleach catalytic activity, such as zinc or aluminum cations, and a sequestrate having defined stability constants for the catalytic and auxiliary metal cations, particularly ethylenediaminetetraacetic acid, ethylenediaminetetra(methylenephosphonic acid) and water-soluble salts thereof. Such catalysts are disclosed in USP 4,430,243.

If desired, the compositions herein can be catalyzed by means of a manganese compound. Such compounds and levels of use are well known in the art and include, for example, the manganese-based catalysts disclosed in USP 5,576,282.

Cobalt bleach catalysts useful herein are known, and are described, for example, in USP 5,597,936; USP 5,595,967. Such cobalt catalysts are readily prepared by known procedures, such as taught for example in USP 5,597,936, and USP 5,595,967.

Compositions herein may also suitably include a transition metal complex of ligands such as bispidones (WO 05/042532 A1) and/or macropolycyclic rigid ligands - abbreviated as "MRLs". As a practical matter, and not by way of limitation, the compositions and processes herein can be adjusted to provide on the order of at least one part per hundred million of the active MRL species in the aqueous washing medium, and will typically provide from about 0.005 ppm to about 25 ppm, from about 0.05 ppm to about 10 ppm, or even from about 0.1 ppm to about 5 ppm, of the MRL in the wash liquor.

Suitable transition-metals in the instant transition-metal bleach catalyst include, for example, manganese, iron and chromium. Suitable MRLs include 5,12-diethyl-1,5,8,12-tetraazabicyclo [6.6.2]hexadecane.

Suitable transition metal MRLs are readily prepared by known procedures, such as taught for example in WO 00/32601, and USP 6,225,464 B1.

Solvents - Suitable solvents include water and other solvents such as lipophilic fluids. Examples of suitable lipophilic fluids include siloxanes, other silicones, hydrocarbons, glycol ethers, glycerine derivatives such as glycerine ethers, perfluorinated amines, perfluorinated and hydrofluoroether solvents, low-volatility nonfluorinated organic solvents, diol solvents, other environmentally-friendly solvents and mixtures thereof.

### Processes of Making Compositions

The compositions of the present invention can be formulated into any suitable form and prepared by any process chosen by the formulator, non-limiting examples of which are described in Applicants' examples and in USP. 4,990,280; U.S. 2003/0087791 A1; U.S. 2003/0087790 A1; U.S. 2005/0003983 A1; U.S. 2004/0048764 A1; USP 4,762,636;USP 6,291,412 B1; U.S. 2005/0227891 A1; EP 1070115A2; USP 5,879,584; USP 5,691,297; USP 5,574,005; USP 5,569,645; USP 5,565,422; USP 5,516,448; USP 5,489,392; and USP 5,486,303.

### Method of Use

The present invention includes a method for cleaning and /or treating a situs *inter alia* a surface or fabric. Such method includes the steps of contacting an embodiment of Applicants' cleaning composition, in neat form or diluted in a wash liquor, with at least a portion of a surface or fabric then optionally rinsing such surface or fabric. The surface or fabric may be subjected to a washing step prior to the aforementioned rinsing step. For purposes of the present invention, washing includes but is not limited to, scrubbing, and mechanical agitation. As will be appreciated by one skilled in the art, the cleaning compositions of the present invention are ideally suited for use in laundry applications. Accordingly, the present invention includes a method for laundering a fabric. The method comprises the steps of contacting a fabric to be laundered with a said cleaning laundry solution comprising at least one embodiment of Applicants' cleaning composition, cleaning additive or mixture thereof. The fabric may comprise most any fabric capable of being laundered in normal consumer use conditions. The solution preferably has a pH of from about 8 to about 10.5. The compositions may be employed at concentrations of from about 500 ppm to about 15,000 ppm in solution. The water temperatures typically range from about 5 °C to about 90 °C. The water to fabric ratio is typically from about 1:1 to about 30:1.

### EXAMPLES

Unless otherwise indicated, materials can be obtained from Aldrich, P.O. Box 2060, Milwaukee, WI 53201, USA.

### Example 1: Production of cellobiose dehydrogenase enzymes

Cellobiose dehydrogenase enzymes can be made as follows:
(a) Cellobiose dehydrogenase (CDH) from *Sclerotium rolfsii* (i) Production of CDH from S. *rolfsii*
   *S. rolfsii* CBS 191.62 can be obtained from the Centraalbureau voor Schimmelcultures, Baarn, The Netherlands.. The organism is maintained through periodic transfer at 25°C on glucose-maltose Sabouraud agar plates. Shaken flask cultures of *S. rolfsii* are grown at 30°C with continuous agitation (110 rpm) in baffled 1000-ml Erlenmeyer flasks containing 500 ml of medium. The medium used for experimental cultures contains 30gl⁻¹ α-cellulose, 20gl⁻¹ Peptone, 20gl⁻¹ Glutamate, 2.5gl⁻¹ NH₄NO₃, 1.5gl⁻¹ MgSO₄.7H₂O, 0.6mll⁻¹ KCl and 0.3mll⁻¹ trace element solution (as per method described by Sachslehner, A. et al. "Production of Hemicellulose Degrading and Cellulose Degrading Enzymes by Various Strains of Sclerotium rolfsii." Appl.Biochem.Biotech 63-65 (1997): 189-201.). The pH is adjusted to 5.0 using phosphoric acid before sterilisation. Several agar plugs cut from the actively growing, outer circumference of a fungal colony growing on Sabouraud plates are used as inocula. Inoculated flasks are cultivated for 7 days. Laboratory-scale cultivations are carried out in a 40-1 stirred tank bioreactor (MBR, Wetzikon, Switzerland) with a working volume of 301. The bioreactor is equipped with instrumentation for measurement and/or control of agitation, temperature, pH, dissolved oxygen concentration and foam. The culture medium is the same medium used for the shaken-flask cultivations and is sterilised in situ at 121°C for 30 min. During the cultivation the temperature is maintained at 25°C and the pH, which is initially adjusted to 5.0, is allowed to float. Agitation is constant at 100 rpm and aeration is held at 1.0 vol. air (fluid vol)⁻¹ min⁻¹. The precultures (10% vol. vol)⁻¹ are 7-day-old shaken-flask cultures grown on the same medium used for the cultivation in the bioreactor.
   After 10 days CDH activity can be detected using standard literature methods described in the assay section (ii) Purification of CDH from *S. rolfsii*
   Mycelium is separated by centrifugation (15Min, 3000 x g), the supernatant is filtered through a glass fiber filter (AP20, 75mm Millipore, Billerica, USA) with pressure filtration and then concentrated using a 10kDa Microza polysulfone ultrafiltration modul (Pall, New York, NY, USA). Crude extracts are further purified using a Q-Sepharose fast flow column from GE Healthcare, installed in an Amersham Pharmacia biotech ÄKTA purifier 900; Pump P-903; Monitor pH/Cond-900; Monitor UV-900; Autosampler Frac-900 (Amersham Biosciences Europe GmbH, Vienna, Austria) , and pre-equilibrated with 50 mM sodium acetate buffer, pH 6.25. Before applying to the column the sample is diluted to a conductivity of below 10mS and the pH is adjusted to 6.25 with sodium carbonate. The proteins are eluted with a linear salt gradient from 0 - 0.5 M NaCl in three column volumes. CDH-active fractions are pooled and concentrated again. The sample is then applied to DEAE Sepharose fast flow column (5x50 cm, Amersham Biosciences, Uppsala, Sweden) pre-equilibrated with 50mM sodium actetate buffer pH 6.25. The proteins are eluted with the sample buffer and with a linear increasing concentration of buffer containing 1M NaCl in eight column volumes. Those fractions with CDH activity are pooled then applied to a Highload Superdex S200 column (26/60 cm, Amersham) equilibrated with 100 mM sodium acetate buffer, pH 4.5, containing 200 mM NaCl, and eluted at 1 ml min⁻¹. Active fractions are again pooled and then applied to a 20 ml Q-Source column (1.6×10 cm, Amersham), which is equilibrated with 20 mM sodium acetate buffer, pH 4.5. The column is eluted by using a linear decreasing buffer gradient (20-1 mM sodium acetate buffer, pH 4.5, in 20 column volumes) The CDH- active fractions are again concentrated and used for further experiments (similar to method described by Baminger, U. et al. "Purification and characterization of cellobiose dehydrogenase from the plant pathogen Sclerotium (Athelia) rolfsii." Applied and Environmental Microbiology 67.4 (2001): 1766-74.).
(b) Production and purification of CDH from *Myriococcum thermophilum*
   *Myriococcum thermophilum* CBS 389.69 can be obtained from the Centraalbureau voor Schimmelcultures, Baarn, The Netherlands. The organism is maintained at 37°C through periodic transfer on glucose-maltose Sabouraud agar plates. Shaken flask cultures are grown at 40°C with continuous agitation (110 rpm) in baffled 1000 mL Erlenmeyer flasks containing 500 mL of culture medium. The medium contains 20 g L⁻¹ a-cellulose, 5 g L⁻¹ peptone, 5 g L⁻¹ yeast extract, 1 gL⁻¹ MgSO₄.7H₂Oand 0.3 mlL⁻¹ trace element solution. The pH is adjusted to pH 6.0 with phosphoric acid before sterilization. Several agar plugs cut from the growing part of a fungal colony are used as inocula. Inoculated flasks are cultivated for up to 10 days until activity of CDH can be measured.
   Both the fermentation and the purification are described in Harreither, W. et al. "Investigation of graphite electrodes modified with cellobiose dehydrogenase from the ascomycete Myriococcum thermophilum." Electroanalysis 19, 2-3 (2007): 172-80.
(c) Production and purification of CDH from Humicola Insolens
   Details of the synthesis and purification of this enzyme can be found in USP 6033981 which is herein incorporated by reference.
(d) Standard enzyme assays.
   Cellobiose Dehydrogenase enzyme activity can be measured using one or more of the following standard literature assays: (i) 2,6-dichlorophenol-indophenol (DCIP) assay
   This measures cellobiose dehydrogenase activity by measuring the time-dependent reduction of 0.3 mM DCIP in 80 mM Na-acetate buffer, pH 4.0, containing 30 mM lactose and 20mM NaF 20µl sample at an absorption of 520 nm and 30°C.
   The extinction coefficient for DCIP at 520 nm and pH 4.0 is 7.3* 10⁻³ M⁻¹ cm⁻¹. One unit of enzyme activity is defined as the amount of enzyme that reduces 1 µmol of DCIP per min under the selected assay conditions (pH 4.0, 30°C). This assay is described in Baminger, U. et al. "A simple assay for measuring cellobiose dehydrogenase activity in the presence of laccase." Journal of Microbiological Methods 35.3 (2002): 253-59.); and/or (ii) Cytochrome c assay Alternatively, CDH activity can be selectively determined by following at 550 nm (30°C) the reduction of 20 µM cytochrome c in 50 mM Na-succinate buffer, pH 3.5, containing 30 mM lactose. The extinction coefficient is 19.6 mM⁻¹ cm⁻¹. This method is described in the literature in Ludwig, R. and D. Haltrich. "Optimisation of cellobiose dehydrogenase production by the fungus Sclerotium (Athelia) rolfsii." Applied Microbiology And Biotechnology 61.1 (2003): 32-39.)

### Examples 2-7 Granular laundry detergent compositions designed for handwashing or top-loading washing machines.

| | 2 | 3 | 4 | 5 | 6 | 7 |
|---|---|---|---|---|---|---|
| | (wt %) | (wt %) | (wt %) | (wt %) | (wt %) | (wt %) |
| Linear alkylbenzenesulfonate | 20 | 22 | 20 | 15 | 20 | 20 |
| C₁₂₋₁₄ Dimethylhydroxyethyl ammonium chloride | 0.7 | 1 | 1 | 0.6 | 0.0 | 0.7 |
| AE3S | 0.9 | 0.0 | 0.9 | 0.0 | 0.0 | 0.9 |
| AE7 | 0.0 | 0.5 | 0.0 | 1 | 3 | 1 |
| Sodium tripolyphosphate | 23 | 30 | 23 | 17 | 12 | 10 |
| Zeolite A | 0.0 | 0.0 | 0.0 | 0.0 | 10 | 0.0 |
| 1.6R Silicate (SiO₂:Na₂O at ratio 1.6:1) | 7 | 7 | 7 | 7 | 7 | 7 |
| Sodium Carbonate | 15 | 14 | 15 | 18 | 15 | 15 |
| Polyacrylate MW 4500 | 1 | 0.0 | 1 | 1 | 1.5 | 1 |
| Carboxy Methyl Cellulose | 1 | 1 | 1 | 1 | 1 | 1 |
| Savinase® 32.89mg/g | 0.1 | 0.07 | 0.1 | 0.1 | 0.1 | 0.1 |
| Natalase® 8.65mg/g | 0.1 | 0.1 | 0.1 | 0.0 | 0.1 | 0.1 |
| Lipex® 18mg/g | 0.03 | 0.07 | 0.3 | 0.1 | 0.07 | 0.4 |
| Fluorescent Brightener 1 | 0.06 | 0.0 | 0.06 | 0.18 | 0.06 | 0.06 |
| Fluorescent Brightener 2 | 0.1 | 0.06 | 0.1 | 0.0 | 0.1 | 0.1 |
| Diethylenetriamine pentaacetic acid | 0.6 | 0.3 | 0.6 | 0.25 | 0.6 | 0.6 |
| MgSO₄ | 1 | 1 | 1 | 0.5 | 1 | 1 |
| Sodium Percarbonate | 0.0 | 5.2 | 0.1 | 0.0 | 0.0 | 0.0 |
| Sodium Perborate Monohydrat | 4.4 | 0.0 | 3.85 | 2.09 | 0.78 | 3.63 |
| NOBS | 1.9 | 0.0 | 1.66 | - | 0.33 | 0.75 |
| TAED | 0.58 | 1.2 | 0.51 | - | 0.015 | 0.28 |
| Sulphonated zinc phthalocyanine | 0.0030 | - | 0.0012 | 0.0030 | 0.0021 | - |
| S-ACMC | 0.1 | 0.06 | - | - | - | |
| Direct Violet 9 | - | - | 0.0003 | 0.0005 | 0.0003 | - |
| Acid Blue 29 | - | - | - | - | - | 0.0003 |
| Cellobiose Dehydrogenase* | 0.003 | 0.002 | 0.006 | 0.004 | 0.003 | 0.02 |
| Sulfate/Moisture | Balance to 100% | Balance to 100% | Balance to 100% | Balance to 100% | Balance to 100% | Balance to 100% |

| | | | | | | |
|---|---|---|---|---|---|---|
| * Refers to enzyme of this invention and the wt% is as active enzyme protein. | | | | | | |

### Examples 8-11 Granular laundry detergent compositions designed for front-loading automatic washing machines.

| | 8 | 9 | 10 | 11 |
|---|---|---|---|---|
| | (wt%) | (wt%) | (wt%) | (wt%) |
| Linear alkylbenzenesulfonate | 8 | 7.1 | 7 | 6.5 |
| AE3S | 0 | 4.8 | 0 | 5.2 |
| Alkylsulfate | 1 | 0 | 1 | 0 |
| AE7 | 2.2 | 0 | 3.2 | 0 |
| C₁₀₋₁₂ Dimethyl hydroxyethylammonium chloride | 0.75 | 0.94 | 0.98 | 0.98 |
| Crystalline layered silicate (δ- Na₂Si₂O₅) | 4.1 | 0 | 4.8 | 0 |
| Zeolite A | 20 | 0 | 17 | 0 |
| Citric Acid | 3 | 5 | 3 | 4 |
| Sodium Carbonate | 15 | 20 | 14 | 20 |
| Silicate 2R (SiO₂:Na₂O at ratio 2:1) | 0.08 | 0 | 0.11 | 0 |
| Soil release agent | 0.75 | 0.72 | 0.71 | 0.72 |
| Acrylic Acid/Maleic Acid Copolymer | 1.1 | 3.7 | 1.0 | 3.7 |
| Carboxymethylcellulose | 0.15 | 1.4 | 0.2 | 1.4 |
| Protease (56.00mg active/g) | 0.37 | 0.4 | 0.4 | 0.4 |
| Termamyl® (21.55mg active/g) | 0.3 | 0.3 | 0.3 | 0.3 |
| Lipex®(18.00mg active/g) | 0.05 | 0.15 | 0.1 | 0.5 |
| Natalase® (8.65mg active/g) | 0.1 | 0.14 | 0.14 | 0.3 |
| TAED | 3.6 | 4.0 | 3.6 | 4.0 |
| Percarbonate | 13 | 13.2 | 13 | 13.2 |
| Na salt of Ethylenediamine-N,N'-disuccinic acid, (S,S) isomer (EDDS) | 0.2 | 0.2 | 0.2 | 0.2 |
| Hydroxyethane di phosphonate (HEDP) | 0.2 | 0.2 | 0.2 | 0.2 |
| MgSO₄ | 0.42 | 0.42 | 0.42 | 0.42 |
| Perfume | 0.5 | 0.6 | 0.5 | 0.6 |
| Suds suppressor agglomerate | 0.05 | 0.1 | 0.05 | 0.1 |
| Soap | 0.45 | 0.45 | 0.45 | 0.45 |
| Sodium sulfate | 22 | 33 | 24 | 30 |
| Sulphonated zinc phthalocyanine (active) | 0.0007 | 0.0012 | 0.0007 | - |
| S-ACMC | 0.01 | 0.01 | - | 0.01 |
| Direct Violet 9 (active) | - | - | 0.0001 | 0.0001 |
| Cellobiose dehydrogenase* | 0.001 | 0.004 | 0.02 | 0.002 |
| Water & Miscellaneous | Balance to 100% | Balance to 100% | Balance to 100% | Balance to 100% |

* Refers to enzyme of this invention and the wt% is as active enzyme protein. Any of the above compositions is used to launder fabrics at a concentration of 10,000 ppm in water, 20-90 °C, and a 5:1 water:cloth ratio. The typical pH is about 10.

### Examples 12-17 Heavy Duty Liquid laundry detergent compositions

| | 12 | 13 | 14 | 15 | 16 | 17 |
|---|---|---|---|---|---|---|
| | (wt%) | (wt%) | (wt%) | (wt%) | (wt%) | (wt%) |
| AES C₁₂₋₁₅ alkyl ethoxy (1.8) sulfate | 11 | 10 | 4 | 6.32 | 6.0 | 8.2 |
| Linear alkyl benzene sulfonate | 4 | 0 | 8 | 3.3 | 4.0 | 3.0 |
| HSAS | 0 | 5.1 | 3 | 0 | 2 | 0 |
| Sodium formate | 1.6 | 0.09 | 1.2 | 0.04 | 1.6 | 1.2 |
| Sodium hydroxide | 2.3 | 3.8 | 1.7 | 1.9 | 2.3 | 1.7 |
| Monoethanolamine | 1.4 | 1.490 | 1.0 | 0.7 | 1.35 | 1.0 |
| Diethylene glycol | 5.5 | 0.0 | 4.1 | 0.0 | 5.500 | 4.1 |
| AE7 | 0.4 | 0.6 | 0.3 | 0.3 | 2 | 0.3 |
| Chelant | 0.15 | 0.15 | 0.11 | 0.07 | 0.15 | 0.11 |
| Citric Acid | 2.5 | 3.96 | 1.88 | 1.98 | 2.5 | 1.88 |
| C₁₂₋₁₄ dimethyl Amine Oxide | 0.3 | 0.73 | 0.23 | 0.37 | 0.3 | 0.225 |
| C₁₂₋₁₈ Fatty Acid | 0.8 | 1.9 | 0.6 | 0.99 | 0.8 | 0.6 |
| Borax | 1.43 | 1.5 | 1.1 | 0.75 | 1.43 | 1.07 |
| Ethanol | 1.54 | 1.77 | 1.15 | 0.89 | 1.54 | 1.15 |
| Ethoxylated (EO₁₅) tetraethylene pentaimine¹ | 0.3 | 0.33 | 0.23 | 0.17 | 0.0 | 0.0 |
| Ethoxylated hexamethylene diamine² | 0.8 | 0.81 | 0.6 | 0.4 | 0.0 | 0.0 |
| 1,2-Propanediol | 0.0 | 6.6 | 0.0 | 3.3 | 0.0 | 0.0 |
| Protease* | 36.4 | 36.4 | 27.3 | 18.2 | 36.4 | 27.3 |
| Mannaway® * | 1.1 | 1.1 | 0.8 | 0.6 | 1.1 | 0.8 |
| Natalase®* | 7.3 | 7.3 | 5.5 | 3.7 | 7.3 | 5.5 |
| Lipex®* | 10 | 3.2 | 0.5 | 3.2 | 2.4 | 3.2 |
| Liquitint® Violet CT (active) | 0.006 | 0.002 | - | - | - | 0.002 |
| S-ACMC | - | - | 0.01 | 0.05 | 0.01 | 0.02 |
| Cellobiose Dehydrogenase** | 0.002 | 0.02 | 0.004 | 0.01 | 0.03 | 0.005 |
| Water, perfume, dyes & other components | Balance | Balance | Balance | Balance | Balance | Balance |

| | | | | | | |
|---|---|---|---|---|---|---|
| * Numbers quoted in mg enzyme/ 100g ** Refers to enzyme of this invention and the wt% is as active enzyme protein. ¹ as described in USP 4,597,898. ² available under the tradename LUTENSIT® from BASF and such as those described in WO 01/05874 | | | | | | |

### Example 18 - ADW dual phase pouch

### Pouch making process:

The composition of Table 1 is introduced in a two compartment layered PVA rectangular base pouch. The dual compartment pouch is made from a Monosol M8630 film as supplied by Chris-Craft Industrial Products. 17.2 g of the particulate composition and 4 g of the liquid composition are placed in the two different compartments of the pouch. The pouch dimensions under 2 Kg load are: length 3.7 cm, width 3.4 cm and height 1.5 cm. The longitudinal/transverse aspect ratio is thus 1.5:3.2 or 1:2.47. The pouch is manufactured using a two-endless surface process, both surfaces moving in continuous horizontal rectilinear motion. According to this process a first web of pouches is prepared by forming and filling a first moving web of open pouches mounted on the first endless surface and closing the first web of open pouches with the second web of filled and sealed pouches moving in synchronism therewith.

**Table 1**

| | 18 |
|---|---|
| | (wt%) |
| Particulate composition | |
| C₁₄ AO | 5 |
| SLF-18 Poly-Tergent® | 5 |
| Hydroxyethane di phosphonate (HEDP) | 1 |
| Termamyl® (21.55mg active/g) | 1.5 |
| Protease (FN3)* | 2 |
| Sodium Percarbonate | 15 |
| Sodium Carbonate | 9 |
| Silicate 2R (SiO₂:Na₂O at ratio 2:1) | 6 |
| Perfume | 0.5 |
| Cellobiose Dehydrogenase* | 0.05 |
| Sodium tripolyphosphate and other adjuncts | Balance to 100% |

| Liquid composition | |
|---|---|
| FN3 Protease (48mg active/ g)* | 0.5 |
| Duramyl Liquid (44 mg active/g) | 0.5 |
| Dye | 0.5 |
| Dipropylene Glycol & other adjuncts | Balance to 100% |

| | |
|---|---|
| * Refers to enzyme of this invention and the wt% is as active enzyme protein. | |

30,000 of the pouches described above and 1,800 L of distilled water at 70°C are introduced in a Dryer Forberg (model FT 5000), the mixture is converted into a slurry, the slurry is then dried and granulated followed by separation with a 850 µm vibrating sieve. The powder which does not pass through the sieve is then ground in a Hammer Mill and reblended with the previously separated fraction. The resulting composition used to manufacture multi-compartment pouches according to the above described manufacturing process.

### Examples 19 -22: Automatic Dishwashing Compositions

| | 19 | 20 | 21 | 22 |
|---|---|---|---|---|
| Example # | (wt %) | (wt %) | (wt %) | (wt %) |
| Silicate 2R (SiO₂:Na₂O at ratio 2:1) | 8.98 | 9.85 | 8.98 | 8.0 |
| Anhydrous Sodium Carbonate | 20.00 | 20.00 | 30.00 | 25.0 |
| Methylglycinediacetic acid, trisodium salt¹ | -- | -- | 6.0 | 0-4.0 |
| Hydroxyethane di phosphonate (HEDP) | 0.23 | 0.23 | 0.23 | 0-0.23 |
| N,N-diacetic glutamic acid tetrasodium salt | -- | -- | 0-6.40 | 0-6.40 |
| Sodium Citrate | 17.00 | 20.00 | -- | -- |
| Co-polymer of acrylate & maleate (45%)² | 7.20 | 10.00 | 5.0-10.0 | 5.0 |
| SLF-18 Poly-Tergent® | 1.00 | 2.00 | 2.0 | 1.0 |
| Polymer³ | 1.43 | 2.86 | -- | -- |
| FN3 Protease (123mg active/g) | 0.19 | 0.28 | 0.16-0.28 | 0.19 |
| Termamyl® (21.55mg active/g) | 0.15 | 0.19 | 0.19-0.28 | 0.12 |
| CT Blend Savinase/Natalase | 0.00 | 0.66 | 0.66 | -- |
| Sodium Perborate Monohydrate | 3.17 | 4.25 | 4.0-4.25 | 3.60 |
| Sodium Sulfate (Anhydrous) | 37.75 | 27.66 | 35.39- 40.70 | 46.98- 49.38 |
| Cellobiose dehydrogenase* | 0.02 | 0.01 | 0.05 | 0.005 |
| Water, perfumes, dye, transition metal bleach catalyst & hydroxyzincite | Balance to 100% | Balance to 100% | Balance to 100% | Balance to 100% |

| | | | | |
|---|---|---|---|---|
| ¹ Sold under tradename Triton® M by BASF, Ludwigshafen, Germany. ² One such suitable polymer would be sold under the tradename Aqualic TL by Nippon Shokubai, Japan. ³ Sold under tradename Alcosperse 725 by Alco Chemical, Tennessee, USA. * Refers to enzyme of this invention and the wt% is as active enzyme protein. | | | | |

### Raw Materials and Notes For Composition Examples 2-22

Linear alkylbenzenesulfonate having an average aliphatic carbon chain length C₁₁-C₁₂ supplied by Stepan, Northfield, Illinois, USA C₁₂₋₁₄ Dimethylhydroxyethyl ammonium chloride, supplied by Clariant GmbH, Sulzbach, Germany AE3S is C₁₂₋₁₅ alkyl ethoxy (3) sulfate supplied by Stepan, Northfield, Illinois, USA AE7 is C₁₂₋₁₅ alcohol ethoxylate, with an average degree of ethoxylation of 7, supplied by Huntsman, Salt Lake City, Utah, USA Sodium tripolyphosphate is supplied by Rhodia, Paris, France Zeolite A is supplied by Industrial Zeolite (UK) Ltd, Grays, Essex, UK 1.6R Silicate is supplied by Koma, Nestemica, Czech Republic Sodium Carbonate is supplied by Solvay, Houston, Texas, USA Polyacrylate MW 4500 is supplied by BASF, Ludwigshafen, Germany Carboxy Methyl Cellulose is Finnfix® BDA supplied by CPKelco, Arnhem, Netherlands Savinase®, Natalase®, Lipex®, Duramyl®, Termamyl®, Mannaway® supplied by Novozymes, Bagsvaerd, Denmark Fluorescent Brightener 1 is Tinopal® AMS, Fluorescent Brightener 2 is Tinopal® CBS-X, Sulphonated zinc phthalocyanine and Direct Violet 9 is Pergasol® Violet BN-Z all supplied by Ciba Specialty Chemicals, Basel, Switzerland Diethylenetriamine pentacetic acid is supplied by Dow Chemical, Midland, Michigan, USA Sodium percarbonate supplied by Solvay, Houston, Texas, USA Sodium perborate is supplied by Degussa, Hanau, Germany NOBS is sodium nonanoyloxybenzenesulfonate, supplied by Eastman, Batesville, Arkansas, USA TAED is tetraacetylethylenediamine, supplied under the Peractive® brand name by Clariant GmbH, Sulzbach, Germany S-ACMC is carboxymethylcellulose conjugated with C.I. Reactive Blue 19, sold by Megazyme, Wicklow, Ireland under the product name AZO-CM-CELLULOSE, product code S-ACMC. Soil release agent is Repel-o-tex® PF, supplied by Rhodia, Paris, France Acrylic Acid/Maleic Acid Copolymer is molecular weight 70,000 and acrylate:maleate ratio 70:30, supplied by BASF, Ludwigshafen, Germany Protease is FN3 supplied by Genencor International, Palo Alto, California, USA Na salt of Ethylenediamine-N,N'-disuccinic acid, (S,S) isomer (EDDS) is supplied by Octel, Ellesmere Port, UK Hydroxyethane di phosphonate (HEDP) is supplied by Dow Chemical, Midland, Michigan, USA Suds suppressor agglomerate is supplied by Dow Coming, Midland, Michigan, USA HSAS is mid-branched alkyl sulfate as disclosed in US 6,020,303 and US 6,060,443 C₁₂₋₁₄ dimethyl Amine Oxide is supplied by Procter & Gamble Chemicals, Cincinnati, Ohio, USA Nonionic is preferably a C₁₂-C₁₃ ethoxylate, preferably with an average degree of ethoxylation of 9.

Protease is supplied by Genencor International, Palo Alto, California, USA Liquitint® Violet CT is supplied by Milliken, Spartanburg, South Carolina, USA

### Other Abbreviations used in Examples

| | | |
|---|---|---|
| Sodium | : | Sodium percarbonate of the nominal formula |
| Percarbonate | | 2Na₂CO₃.3H₂O₂ |
| C₁₄AO | : | tetradecyl dimethyl amine oxide |

The dimensions and values disclosed herein are not to be understood as being strictly limited to the exact numerical values recited. Instead, unless otherwise specified, each such dimension is intended to mean both the recited value and functionally equivalent range surrounding that value. For example, a dimension disclosed as "40 mm" is intended to mean "about 40 mm".

All documents cited in the Detailed Description of the Invention are, in relevant part, incorporated herein by reference; the citation of any document is not to be construed as an admission that it is prior art with respect to the present invention. To the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A composition comprising one or more adjunct ingredients, said one or more adjunct ingredients are selected from the group consisting of surfactants, builders, chelating agents, dye transfer inhibiting agents, dispersants, additional enzymes, and enzyme stabilizers, catalytic materials, bleaching agents, polymeric dispersing agents, clay soil removal/anti-redeposition agents, brighteners, suds suppressors, dyes, perfumes, structure elasticizing agents, fabric softeners, carriers, hydrotropes, processing aids, solvents, pigments, hueing agents, photobleaches, structurants, and mixtures thereof, preferably said one or more adjunct ingredients comprises an enzyme, preferably said enzyme is selected from the group consisting of peroxidases, proteases, lipases, phospholipases, cellobiohydrolases, esterases, cutinases, pectinases, mannanases, pectate lyases, keratinases, reductases, oxidases, phenoloxidases, lipoxygenases, ligninases, pullulanases, tannases, pentosanases, glucanases, arabinosidases, hyaluronidase, chondroitinase, laccases, amylases, or mixtures thereof, more preferably said enzyme is selected from the group consisting of:
a.) lipases, such as lipases having greater than 90% amino acid sequence identity to SEQ. ID 8;
b.) alpha-amylases, such as alpha-amylases having greater than 90% amino acid sequence identity to SEQ. ID 9;
c.) endoglucanases, such as endoglucanases having greater than 90% amino acid sequence identity to SEQ. ID 10;
d.) proteases, preferably metalloproteases, neutral or alkaline serine proteases, preferably a subtilisins, preferably a subtilisin derived from *Bacillus lentus, B. alkalophilus, B. subtilis, B. amyloliquefaciens*, and mixtures thereof; and
e.) mixtures thereof; and a cellobiose dehydrogenase enzyme said composition comprising, based on total composition weight, 0% weight % cellulase or said composition having a mass ratio of cellobiose dehydrogenase enzyme:cellulase of from 0.01 to 1,000,000, preferably from 0.02 to 500,000, more preferably from 0.05 to 200,000 or most preferably from 0.1 to 10,000; optionally said composition comprises a material selected from the group consisting of :
a) oligosaccharides selected from the group consisting of cello-oligosaccharides, preferably a cello-oligosaccharides selected from the group consisting of cellobiose, cello-triose, cello-tetraose, cello-pentaose, cello-hexaose, cellodextrin and mixtures thereof, lactose, maltose, xylobiose and mixtures thereof;
b) amorphous cellulose;
c) cellulosic polymers preferably carboxy methyl cellulose; and
d) mixtures thereof.

2. The composition of Claim 1 wherein said cellobiose dehydrogenase has a minimum of 60% amino acid sequence identity with the wild-type cellobiose dehydrogenases derived from a microorganism selected from the group consisting of *Myceliophtore thermophila, Phanerochaete chrysosporium, Trametes versicolor, Grifola frondosa, Neosartorya fischeri, Humicola insolens, Sclerotium rolfsii, Myriococcum thermophilum* and combinations thereof.

3. A composition according to any one of the preceding claims wherein said cellobiose dehydrogenase has a minimum of 70% amino acid sequence identity with any enzyme having a sequence corresponding to a sequence ID selected from the group consisting of SEQ. ID Nos. 1, 2, 3, 4, 5, 6, or 7, preferably said cellobiose dehydrogenase enzyme has a minimum of 85% amino acid sequence identity with a sequence corresponding to a sequence ID selected from the group consisting of SEQ. ID Nos. 1, 2, or amino acids 22 to 785 of SEQ. ID No. 3.

4. A composition according to any one of the preceding claims wherein said cellobiose dehydrogenase enzyme comprises a carbohydrate binding module, preferably a cellulose-binding domain.

5. A composition according to any one of the preceding claims wherein said cellobiose dehydrogenase comprises a cellobiose dehydrogenase domain and a cellobiohydrolase domain.

6. A composition according to any one of the preceding claims, said composition comprising a material selected from the group consisting of a surfactant, a polymer, a builder and mixtures thereof, preferably said surfactant being selected from the group of:
a.) anionic surfactants, preferably an anionic surfactant selected from the group consisting of linear alkylbenzene-sulfonate (LAS), alcohol ethoxysulfate (AES), mid-branched alkyl sulfates (HSAS) and mixtures thereof;
b.) non ionic surfactants preferably alcohol ethoxylates, said alcohol ethoxylates, preferably having a chain length of from 1 to 18 carbons;
c.) amine oxides; and
d.) mixtures thereof; preferably said polymer being selected from the group consisting of
a.) polyacrylates;
b.) maleic/acrylic acid copolymers;
c.) cellulose-derived polymers, preferably cellulose-derived polymers selected from the group consisting of carboxymethylcellulose, methyl hydroxyethylcellulose and mixtures thereof;
d.) polyethyleneimine polymers;
e.) soil release polymers, preferably co-polymers comprising two or more monomers selected from the group consisting of sulfo-isophthalate, ethylene terephthalate, oxyethyleneterephthalate and mixtures thereof; and
f.) mixtures thereof; preferably said builder being selected from the group consisting of
a.) citric acid;
b.) C₁₂-C₁₈ fatty acids;
c.) aluminosilicates, in one aspect a zeolite selected from the group consisting of Zeolite A, Zeolite X , Zeolite Y and mixtures thereof;
d.) sodium tripolyphosphate; and
e.) mixtures thereof; preferably said composition comprising, based on total product weight, less than 15%, preferably less than 10%, more preferably less than 8% or most preferably from 0.01 % to 7% of said builder.

7. A composition according to any one of the preceding claims, comprising a material selected from the group consisting of a photobleach, a fabric hueing agent and mixtures thereof, preferably said photobleach being selected from the group consisting of
a.) xanthene dyes and mixtures thereof;
b.) sulfonated zinc phthalocyanine, sulfonated aluminium phthalocyanine, Eosin Y, Phoxine B, Rose Bengal, C.I. Food Red 14 and mixtures thereof;
c.) water soluble phthalocyanine; and
d.) mixtures thereof; and said fabric hueing agent preferably being selected from the group consisting of
a.) dyes, preferably said dyes being selected from the group consisting of small molecule dyes, polymeric dyes and mixtures thereof;
b.) dye-clay conjugates comprising at least one cationic/basic dye and a smectite clay; and
c.) mixtures thereof; and mixtures thereof.

8. A composition according to any one of the preceding claims" said composition comprising a bleaching agent selected from one or more of the following:
a) a source of hydrogen peroxide;
b) a bleach activator, preferably tetraacetyl ethylene diamine (TAED) and /or nonanoyloxybenzene sulphonate (NOBS);
c) a pre-formed peracid;
d) a diacyl peroxide;
e) a tetraacyl peroxide; and
f) mixtures thereof; and/or a bleach catalyst that is capable of accepting an oxygen atom from a peroxyacid and transferring the oxygen atom to an oxidizeable substrate.

9. A composition according to any one of the preceding claims, comprising an encapsulated enzyme and/or an enzyme prill, preferably said encapsulated enzyme and/or enzyme prill comprising a cellobiose dehydrogenase enzyme.

10. A method of treating and/or cleaning a surface or fabric comprising the steps of optionally washing and/or rinsing said surface or fabric, contacting said surface or fabric with a composition according to any preceding claim, then optionally washing and/or rinsing said surface or fabric.


SEQUENCE LISTING

<110>  Procter & Gamble International Operations SA

<120>  CLEANING AND/OR TREATMENT COMPOSITIONS

<130>  CM3252F

<160>  10

<170>  PatentIn version 3.3

<210>  1
<211>  828
<212>  PRT
<213>  Myceliophtore thermophila

<400>  1

Met Arg Thr Ser Ser Arg Leu Ile Gly Ala Leu Ala Ala Ala Leu Leu
1               5                   10                  15

Pro Ser Ala Leu Ala Gln Asn Asn Ala Pro Val Thr Phe Thr Asp Pro
            20                  25                  30

Asp Ser Gly Ile Thr Phe Asn Thr Trp Gly Leu Ala Glu Asp Ser Pro
        35                  40                  45

Gln Thr Lys Gly Gly Phe Thr Phe Gly Val Ala Leu Pro Ser Asp Ala
    50                  55                  60

Leu Thr Thr Asp Ala Lys Glu Phe Ile Gly Tyr Leu Lys Cys Ala Arg
65                  70                  75                  80

Asn Asp Glu Ser Gly Trp Cys Gly Val Ser Leu Gly Gly Pro Met Thr
                85                  90                  95

Asn Ser Leu Leu Ile Ala Ala Trp Pro His Glu Asp Thr Val Tyr Thr
            100                 105                 110

Ser Leu Arg Phe Ala Thr Gly Tyr Ala Met Pro Asp Val Tyr Gln Gly
        115                 120                 125

Asp Ala Glu Ile Thr Gln Val Ser Ser Ser Val Asn Ser Thr His Phe
    130                 135                 140

Ser Leu Ile Phe Arg Cys Glu Asn Cys Leu Gln Trp Ser Gln Ser Gly
145                 150                 155                 160

Ala Thr Gly Gly Ala Ser Thr Ser Asn Gly Val Leu Val Leu Gly Trp
                165                 170                 175

Val Gln Ala Phe Ala Asp Pro Gly Asn Pro Thr Cys Pro Asp Gln Ile
            180                 185                 190

Thr Leu Glu Gln His Asp Asn Gly Met Gly Ile Trp Gly Ala Gln Leu

        195                 200                 205

Asn Ser Asp Ala Ala Ser Pro Ser Tyr Thr Glu Trp Ala Ala Gln Ala
    210                 215                 220

Thr Lys Thr Val Thr Gly Asp Cys Gly Gly Pro Thr Glu Thr Ser Val
225                 230                 235                 240

Val Gly Val Pro Val Pro Thr Gly Val Ser Phe Asp Tyr Ile Val Val
                245                 250                 255

Gly Gly Gly Ala Gly Gly Ile Pro Ala Ala Asp Lys Leu Ser Glu Ala
            260                 265                 270

Gly Lys Ser Val Leu Leu Ile Glu Lys Gly Phe Ala Ser Thr Ala Asn
        275                 280                 285

Thr Gly Gly Thr Leu Gly Pro Glu Trp Leu Glu Gly His Asp Leu Thr
    290                 295                 300

Arg Phe Asp Val Pro Gly Leu Cys Asn Gln Ile Trp Val Asp Ser Lys
305                 310                 315                 320

Gly Ile Ala Cys Glu Asp Thr Asp Gln Met Ala Gly Cys Val Leu Gly
                325                 330                 335

Gly Gly Thr Ala Val Asn Ala Gly Leu Trp Phe Lys Pro Tyr Ser Leu
            340                 345                 350

Asp Trp Asp Tyr Leu Phe Pro Ser Gly Trp Lys Tyr Lys Asp Val Gln
        355                 360                 365

Pro Ala Ile Asn Arg Ala Leu Ser Arg Ile Pro Gly Thr Asp Ala Pro
    370                 375                 380

Ser Thr Asp Gly Lys Arg Tyr Tyr Gln Gln Gly Phe Asp Val Leu Ser
385                 390                 395                 400

Lys Gly Leu Ala Gly Gly Gly Trp Thr Ser Val Thr Ala Asn Asn Ala
                405                 410                 415

Pro Asp Lys Lys Asn Arg Thr Phe Ser His Ala Pro Phe Met Phe Ala
            420                 425                 430

Gly Gly Glu Arg Asn Gly Pro Leu Gly Thr Tyr Phe Gln Thr Ala Lys
        435                 440                 445

Lys Arg Ser Asn Phe Lys Leu Trp Leu Asn Thr Ser Val Lys Arg Val
    450                 455                 460

Ile Arg Gln Gly Gly His Ile Thr Gly Val Glu Val Glu Pro Phe Arg

465                 470                 475                 480

Asp Gly Gly Tyr Gln Gly Ile Val Pro Val Thr Lys Val Thr Gly Arg
                485                 490                 495

Val Ile Leu Ser Ala Gly Thr Phe Gly Ser Ala Lys Ile Leu Leu Arg
            500                 505                 510

Ser Gly Ile Gly Pro Asn Asp Gln Leu Gln Val Val Ala Ala Ser Glu
        515                 520                 525

Lys Asp Gly Pro Thr Met Ile Ser Asn Ser Ser Trp Ile Asn Leu Pro
    530                 535                 540

Val Gly Tyr Asn Leu Asp Asp His Leu Asn Thr Asp Thr Val Ile Ser
545                 550                 555                 560

His Pro Asp Val Val Phe Tyr Asp Phe Tyr Glu Ala Trp Asp Asn Pro
                565                 570                 575

Ile Gln Ser Asp Lys Asp Ser Tyr Leu Asn Ser Arg Thr Gly Ile Leu
            580                 585                 590

Ala Gln Ala Ala Pro Asn Ile Gly Pro Met Phe Trp Glu Glu Ile Lys
        595                 600                 605

Gly Ala Asp Gly Ile Val Arg Gln Leu Gln Trp Thr Ala Arg Val Glu
    610                 615                 620

Gly Ser Leu Gly Ala Pro Asn Gly Lys Thr Met Thr Met Ser Gln Tyr
625                 630                 635                 640

Leu Gly Arg Gly Ala Thr Ser Arg Gly Arg Met Thr Ile Thr Pro Ser
                645                 650                 655

Leu Thr Thr Val Val Ser Asp Val Pro Tyr Leu Lys Asp Pro Asn Asp
            660                 665                 670

Lys Glu Ala Val Ile Gln Gly Ile Ile Asn Leu Gln Asn Ala Leu Lys
        675                 680                 685

Asn Val Ala Asn Leu Thr Trp Leu Phe Pro Asn Ser Thr Ile Thr Pro
    690                 695                 700

Arg Gln Tyr Val Asp Ser Met Val Val Ser Pro Ser Asn Arg Arg Ser
705                 710                 715                 720

Asn His Trp Met Gly Thr Asn Lys Ile Gly Thr Asp Asp Gly Arg Lys
                725                 730                 735

Gly Gly Ser Ala Val Val Asp Leu Asn Thr Lys Val Tyr Gly Thr Asp

740                    745                    750

Asn Leu Phe Val Ile Asp Ala Ser Ile Phe Pro Gly Val Pro Thr Thr
        755                 760                 765

Asn Pro Thr Ser Tyr Ile Val Thr Ala Ser Glu His Ala Ser Ala Arg
    770                 775                 780

Ile Leu Ala Leu Pro Asp Leu Thr Pro Val Pro Lys Tyr Gly Gln Cys
785                 790                 795                 800

Gly Gly Arg Glu Trp Ser Gly Ser Phe Val Cys Ala Asp Gly Ser Thr
                805                 810                 815

Cys Gln Met Gln Asn Glu Trp Tyr Ser Gln Cys Leu
            820                 825

<210>  2
<211>  771
<212>  PRT
<213>  Sclerotium rolfsii

<400>  2

Met Leu Ser Arg Leu Val Leu Asn Leu Leu Ala Ile Thr Val Ile Gly
1               5                   10                  15

Val Phe Gly Gln Ser Ser Ser Ser Tyr Thr Asp Asn Gly Ile Asn Phe
            20                  25                  30

Gln Gly Ile Thr Asp Pro Thr Tyr Gly Val Thr Tyr Gly Ala Val Phe
        35                  40                  45

Pro Pro Ala Ser Val Asp Ser Asp Glu Phe Ile Gly Glu Ile Ala Ala
    50                  55                  60

Pro Val Ala Ala Lys Trp Ile Gly Leu Ser Leu Gly Gly Ala Met Ile
65                  70                  75                  80

Asn Asn Leu Leu Ile Val Ala Trp Pro Asn Asn Asn Glu Ile Val Phe
                85                  90                  95

Ser Ser Arg Tyr Thr Thr Gly Tyr Val Leu Pro Thr Ile Tyr Ser Gly
            100                 105                 110

Pro Lys Ile Thr Thr Ile Ser Ser Ser Val Asn Ser Thr His Trp Lys
        115                 120                 125

Trp Ile Tyr Arg Cys Gln Asn Cys Thr Thr Trp Ser Gly Gly Ser Leu
    130                 135                 140

Ala Ala Asn Gly Ser Ala Val Trp Ala Trp Ala Tyr Ser Ser Ala Ala
145                 150                 155                 160

Val Asp Thr Pro Ser Ser Pro Ser Ser Ser Phe Asp Glu His Thr Asp
                165                 170                 175

Phe Gly Phe Phe Gly Glu Ile Thr Ser Asn Ala His Val Ser Gln Ser
            180                 185                 190

Val Tyr Glu Gln Tyr Leu Thr Gly Thr Gly Val Thr Ser Thr Ser Ser
        195                 200                 205

Ser Thr Ser Thr Thr Thr Ser Thr Ser Thr Thr Thr Ser Thr Ser Ser
    210                 215                 220

Ala Pro Thr Val Ser Ala Thr Pro Tyr Asp Tyr Ile Ile Val Gly Ala
225                 230                 235                 240

Gly Pro Ala Gly Ile Ile Ala Ala Asp Arg Leu Ser Glu Ala Gly Lys
                245                 250                 255

Lys Val Leu Leu Leu Glu Arg Gly Gly Pro Ser Thr Lys Glu Thr Gly
            260                 265                 270

Gly Thr Tyr Thr Ala Pro Trp Ala Ala Ser Ser Gly Leu Thr Lys Phe
        275                 280                 285

Asp Ile Pro Gly Val Phe Glu Ser Leu Phe Thr Asp Ser Asn Ser Phe
    290                 295                 300

Trp Trp Cys Lys Asp Ile Thr Val Phe Ala Gly Cys Leu Thr Gly Gly
305                 310                 315                 320

Gly Thr Ala Ile Asn Gly Ala Leu Tyr Trp Tyr Pro Thr Asp Leu Asp
                325                 330                 335

Phe Ser Thr Ala Asn Gly Trp Pro Ser Ser Trp Thr Ala His Gln Lys
            340                 345                 350

Tyr Thr Asp Leu Val Ser Ser Arg Leu Pro Ser Ser Asp His Pro Ser
        355                 360                 365

Thr Asp Gly Lys Arg Tyr Leu Glu Gln Ser Ala Ala Val Val Ala Gln
    370                 375                 380

Leu Leu Asn Gly Gln Gly Tyr Arg Asn Lys Thr Ile Asn Asn Ala Pro
385                 390                 395                 400

Asn Ala Lys Asp His Val Tyr Gly Tyr Ser Ala Phe Asp Phe Leu Asn
                405                 410                 415

Gly Lys Arg Ala Gly Pro Val Ala Thr Tyr Leu Gln Thr Ala Lys Thr
            420                 425                 430

Arg Asn Asn Phe His Phe Lys Asn Tyr Val Leu Val Ser Asn Val Val
        435                 440                 445

Arg Asn Gly Ser Thr Ile Thr Gly Val Lys Thr Asn Asp Thr Ser Leu
    450                 455                 460

Gly Pro Asn Gly Val Ile Pro Leu Thr Lys Asn Gly Arg Val Ile Leu
465                 470                 475                 480

Ser Ala Gly Ser Leu Ser Ser Pro Arg Ile Leu Phe Gln Ser Gly Ile
                485                 490                 495

Gly Pro Thr Asp Met Leu Thr Leu Val Gln Asn Asn Pro Thr Ala Ser
            500                 505                 510

Ala Asn Leu Pro Ser Gln Ser Gln Trp Ile His Leu Pro Val Gly Tyr
        515                 520                 525

Asn Val Ala Asp Ala Pro Ser Ile Asn Phe Val Phe Thr His Pro Ser
    530                 535                 540

Ile Asp Ala Tyr Asp Asn Trp Ala Asn Val Trp Thr Asn Pro Arg Ser
545                 550                 555                 560

Thr Asp Ala Glu Gln Tyr Leu Lys Asn Gln Ser Gly Val Leu Ala Ala
                565                 570                 575

Ser Ser Pro Lys Leu Asn Phe Trp Arg Ala Tyr Gly Gly Ser Asp Gly
            580                 585                 590

Arg Thr Arg Trp Met Gln Gly Thr Val Arg Pro Gly Ala Ala Ser Ile
        595                 600                 605

Asn Thr Thr Tyr Asn Tyr Asn Ala Ser Gln Ile Phe Thr Ile Thr Thr
    610                 615                 620

Tyr Val Ser Thr Gly Ile Thr Ser Arg Gly Arg Ile Gly Val Thr Ser
625                 630                 635                 640

Ser Leu Asn Val Leu Pro Leu Val Asn Pro Trp Leu Val Asp Pro Val
                645                 650                 655

Asp Glu Lys Val Leu Thr Gln Ser Leu Glu Asp Leu Val Ser Asn Met
            660                 665                 670

Lys Ser Val Pro Gly Leu Thr Met Ile Met Pro Asp Asn Thr Thr Ser
        675                 680                 685

Ile Ala Asp Tyr Val Lys Asn Tyr Asp Arg Ala Ser Met Asn Ser Asn
    690                 695                 700

His Trp Val Gly Ser Asn Lys Ile Ala Ser Asn Ala Thr Glu Gly Val
705                 710                 715                 720

Val Asp Glu His Thr Lys Val Phe Arg Thr Asp Asn Leu Phe Ile Val
                725                 730                 735

Asp Ala Ser Ile Ile Pro Ser Leu Pro Met Gly Asn Pro Gln Gly Ala
            740                 745                 750

Leu Met Ser Ala Ala Glu Gln Ala Val Ala Lys Ile Leu Ala Leu Ala
        755                 760                 765

Gly Gly Pro
    770

<210>  3
<211>  785
<212>  PRT
<213>  Humicola insolens

<400>  3

Met Lys Phe Leu Gly Arg Ile Gly Ala Thr Ala Leu Ala Ala Ser Leu
1               5                   10                  15

Tyr Leu Thr Ser Gly Ala Ala Gln Ala Thr Gly Asp Ala Tyr Thr Asp
            20                  25                  30

Ser Glu Thr Gly Ile Lys Phe Gln Thr Trp Ser Pro Asp Pro Gln Phe
        35                  40                  45

Thr Phe Gly Leu Ala Leu Pro Pro Asp Ala Leu Glu Lys Asp Ala Thr
    50                  55                  60

Glu Tyr Ile Gly Leu Leu Arg Cys Thr Arg Ala Asp Pro Ser Asp Pro
65                  70                  75                  80

Gly Tyr Cys Gly Leu Ser His Gly Gln Val Gly Gln Met Thr Gln Ser
                85                  90                  95

Leu Leu Leu Val Ala Trp Ala Tyr Glu Asn Gln Val Tyr Thr Ser Phe
            100                 105                 110

Arg Tyr Ala Thr Gly Tyr Thr Leu Pro Gly Leu Tyr Thr Gly Asn Ala
        115                 120                 125

Lys Leu Thr Gln Leu Ser Val Asn Ile Thr Asp Thr Ser Phe Glu Leu
    130                 135                 140

Ile Tyr Arg Cys Glu Asn Cys Phe Ser Trp Glu His Glu Gly Ser Thr
145                 150                 155                 160

Gly Ser Ser Ser Thr Ser Gln Gly Tyr Leu Val Leu Gly Arg Ala Ser
                165                 170                 175

Ala Arg Arg Gly Val Val Gly Pro Thr Cys Pro Asp Thr Ala Thr Phe
            180                 185                 190

Gly Phe His Asp Asn Gly Phe Gly Gln Trp Gly Val Gly Leu Glu Asn
        195                 200                 205

Ala Val Ser Glu Gln Tyr Ser Glu Trp Ala Ser Leu Pro Gly Leu Thr
    210                 215                 220

Val Glu Thr Thr Cys Glu Gly Ser Gly Pro Gly Glu Ala Gln Cys Val
225                 230                 235                 240

Pro Ala Pro Glu Glu Thr Tyr Asp Tyr Ile Val Val Gly Ala Gly Ala
                245                 250                 255

Gly Gly Ile Pro Val Ala Asp Lys Leu Ser Glu Ala Gly His Lys Val
            260                 265                 270

Leu Leu Ile Glu Lys Gly Pro Pro Ser Thr Gly Arg Trp Gln Gly Thr
        275                 280                 285

Met Lys Pro Glu Trp Leu Glu Gly Thr Asp Leu Thr Arg Phe Asp Val
    290                 295                 300

Pro Gly Leu Cys Asn Gln Ile Trp Val Asp Ser Ala Gly Ile Ala Cys
305                 310                 315                 320

Thr Asp Thr Asp Gln Met Ala Gly Cys Val Leu Gly Gly Gly Thr Ala
                325                 330                 335

Val Asn Ala Gly Leu Trp Trp Lys Pro Ile Asp Leu Asp Trp Asp Glu
            340                 345                 350

Asn Phe Pro Glu Gly Trp His Ser Gln Asp Leu Ala Ala Ala Thr Glu
        355                 360                 365

Arg Val Phe Glu Arg Ile Pro Gly Thr Trp His Pro Ser Met Asp Gly
    370                 375                 380

Lys Leu Tyr Arg Asp Glu Gly Tyr Lys Val Leu Ser Ser Gly Leu Ala
385                 390                 395                 400

Glu Ser Gly Trp Lys Glu Val Val Ala Asn Glu Val Pro Asn Glu Lys
                405                 410                 415

Asn Arg Thr Phe Ala His Thr His Phe Met Phe Ala Gly Gly Glu Arg
            420                 425                 430

Asn Gly Pro Leu Ala Thr Tyr Leu Val Ser Ala Asp Ala Arg Glu Asn
        435                 440                 445

Phe Ser Leu Trp Thr Asn Thr Ala Val Arg Arg Ala Val Arg Thr Gly
    450                 455                 460

Gly Lys Val Thr Gly Val Glu Leu Glu Cys Leu Thr Asp Gly Gly Tyr
465                 470                 475                 480

Ser Gly Ile Val Lys Leu Asn Glu Gly Gly Gly Val Ile Phe Ser Ala
                485                 490                 495

Gly Ala Phe Gly Ser Ala Lys Leu Leu Phe Arg Ser Gly Ile Gly Pro
            500                 505                 510

Glu Asp Gln Leu Arg Val Val Ala Ser Ser Lys Asp Gly Glu Asp Phe
        515                 520                 525

Ile Asp Glu Lys Asp Trp Ile Lys Leu Pro Val Gly Tyr Asn Leu Ile
    530                 535                 540

Asp His Leu Asn Thr Asp Leu Ile Leu Thr His Pro Asp Val Val Phe
545                 550                 555                 560

Tyr Asp Phe Tyr Glu Ala Trp Thr Thr Pro Ile Glu Ala Asp Lys Gln
                565                 570                 575

Leu Tyr Leu Glu Gln Arg Ser Gly Ile Leu Ala Gln Ala Ala Pro Asn
            580                 585                 590

Ile Gly Pro Met Met Trp Glu Gln Val Thr Pro Ser Asp Gly Ile Thr
        595                 600                 605

Arg Gln Phe Gln Trp Thr Ala Arg Val Glu Gly Asp Ser Arg Phe Thr
    610                 615                 620

Asn Ser Ser His Ala Met Thr Leu Ser Gln Tyr Leu Gly Arg Gly Val
625                 630                 635                 640

Val Ser Arg Gly Arg Ala Thr Ile Thr Gln Gly Leu Val Thr Thr Val
                645                 650                 655

Ala Glu His Pro Tyr Leu His Asn Ala Gly Asp Lys Glu Ala Val Ile
            660                 665                 670

Gln Gly Ile Lys Asn Leu Ile Glu Ser Leu Asn Val Ile Pro Asn Ile
        675                 680                 685

Thr Trp Val Leu Pro Pro Pro Gly Ser Thr Val Glu Glu Tyr Val Asp
    690                 695                 700

Ser Leu Leu Val Ser Ala Ser Ala Arg Arg Ser Asn His Trp Met Gly
705                 710                 715                 720

Thr Ala Lys Leu Gly Thr Asp Asp Gly Arg Tyr Gly Gly Thr Ser Val
                725                 730                 735

Val Asp Leu Asp Thr Lys Val Tyr Gly Thr Asp Asn Leu Phe Val Val
            740                 745                 750

Asp Ala Ser Ile Phe Pro Gly Met Ser Thr Gly Asn Pro Ser Ala Met
        755                 760                 765

Ile Val Ile Ala Ala Glu Gln Ala Ala Glu Arg Ile Leu Lys Leu Arg
    770                 775                 780

Lys
785

<210>  4
<211>  768
<212>  PRT
<213>  Trametes versicolor

<400>  4

Met Lys Phe Lys Ser Leu Leu Leu Ser Leu Leu Pro Leu Val Gly Ser
1               5                   10                  15

Val Tyr Ser Gln Val Ala Ala Pro Tyr Val Asp Ser Gly Asn Gly Phe
            20                  25                  30

Val Phe Asp Gly Val Thr Asp Pro Val His Ser Val Thr Tyr Gly Ile
        35                  40                  45

Val Leu Pro Gln Ala Ser Thr Ser Thr Glu Phe Ile Gly Glu Phe Val
    50                  55                  60

Ala Pro Asn Glu Ala Gln Trp Ile Gly Leu Ala Leu Gly Gly Ala Met
65                  70                  75                  80

Ile Gly Asn Leu Leu Leu Val Ala Trp Pro Asn Gly Asn Lys Ile Val
                85                  90                  95

Ser Ser Pro Arg Tyr Ala Thr Gly Tyr Thr Leu Pro Ala Ala Tyr Ala
            100                 105                 110

Gly Pro Thr Ile Thr Gln Leu Pro Ser Ser Ser Val Asn Ser Thr His
        115                 120                 125

Trp Lys Phe Val Phe Arg Cys Gln Asn Cys Thr Ala Trp Asn Gly Gly
    130                 135                 140

Ser Ile Asp Pro Ser Gly Thr Gly Val Phe Ala Trp Ala Phe Ser Asn
145                 150                 155                 160

Val Ala Val Asp Asp Pro Ser Asp Pro Asn Ser Ser Phe Ala Glu His
                165                 170                 175

Thr Asp Phe Gly Phe Phe Gly Ile Asn Phe Pro Asp Ala Gln Ser Ser
            180                 185                 190

Asn Tyr Gln Asn Tyr Leu Ala Gly Asn Ala Gly Thr Pro Pro Pro Thr
        195                 200                 205

Ser Val Pro Ser Gly Pro Ser Ser Thr Thr Thr Thr Thr Gly Pro Thr
    210                 215                 220

Ala Thr Ala Thr Pro Phe Asp Tyr Ile Val Val Gly Ala Gly Pro Gly
225                 230                 235                 240

Gly Leu Val Thr Ala Asp Arg Leu Ser Glu Ala Gly Lys Lys Val Leu
                245                 250                 255

Leu Leu Glu Arg Gly Gly Pro Ser Thr Ala Glu Thr Gly Gly Thr Tyr
            260                 265                 270

Asp Ala Thr Trp Ala Lys Ser Ala Asn Leu Thr Lys Phe Asp Val Pro
        275                 280                 285

Gly Leu Phe Glu Thr Leu Phe Thr Asp Thr Asn Pro Phe Trp Trp Cys
    290                 295                 300

Lys Asp Thr Asn Phe Phe Ala Gly Cys Leu Leu Gly Gly Gly Thr Ser
305                 310                 315                 320

Val Asn Gly Ala Leu Tyr Trp Tyr Pro Asn Ser Arg Asp Phe Ser Thr
                325                 330                 335

Ala Ser Gly Trp Pro Ser Ser Trp Ser Asn His Gln Pro Phe Thr Asp
            340                 345                 350

Lys Leu Lys Gln Arg Leu Pro Ser Thr Asp His Pro Ser Ala Asp Gly
        355                 360                 365

Gln Arg Tyr Leu Glu Gln Ser Ala Thr Val Val Gln Gln Leu Leu Ser
    370                 375                 380

Gly Gln Gly Tyr Ser Gln Ile Thr Ile Asn Gly Asn Pro Asp Ser Lys
385                 390                 395                 400

Asp His Val Phe Gly Phe Ser Ala Phe Asp Phe Leu Asn Gly Gln Arg
                405                 410                 415

Ala Gly Ser Val Ala Thr Tyr Phe Glu Thr Ala Leu Ala Arg Lys Asn
            420                 425                 430

Phe Val Tyr Lys Asp Asn Val Leu Val Thr Gln Val Ile Arg Asn Gly
        435                 440                 445

Ser Thr Ile Leu Gly Val Arg Thr Asn Asp Asn Thr Leu Gly Pro Asp
    450                 455                 460

Gly Val Val Pro Leu Asn Pro Asn Gly Arg Val Ile Leu Ser Gly Gly
465                 470                 475                 480

Ser Phe Gly Thr Pro Arg Ile Leu Phe Gln Ser Gly Ile Gly Pro Thr
                485                 490                 495

Asp Met Leu Gln Thr Val Gln Ser Asn Ala Gln Ala Ala Ala Asn Leu
            500                 505                 510

Pro Pro Gln Ser Glu Trp Ile Asp Leu Pro Val Gly Gln Ser Val Ser
        515                 520                 525

Asp Asn Pro Ser Ile Asn Leu Val Phe Thr His Pro Ser Ile Asp Ala
    530                 535                 540

Tyr Asp Asn Trp Ala Asp Val Trp Ser Asn Pro Arg Pro Ala Asp Ala
545                 550                 555                 560

Gln Gln Tyr Leu Gln Ser Arg Ser Gly Val Leu Ala Gly Ala Ser Leu
                565                 570                 575

Lys Leu Asn Phe Trp Arg Ala Tyr Gly Gly Ser Asp Gly Ile Thr Gly
            580                 585                 590

Tyr Ala Gln Gly Thr Val Arg Pro Gly Ala Ala Ser Val Asn Thr Ser
        595                 600                 605

Val Ala Tyr Asn Ala Ser Glu Ile Phe Thr Thr Thr Leu Tyr Leu Ser
    610                 615                 620

Asn Gly Ile Gln Ser Arg Gly Arg Ile Gly Val Asp Ala Thr Leu Asn
625                 630                 635                 640

Ala Lys Ala Leu Val Asn Pro Trp Leu Thr Asn Ser Val Asp Lys Thr
                645                 650                 655

Val Leu Leu Gln Ala Leu His Asp Val Thr Ser Thr Met Lys Asn Val
            660                 665                 670

Ser Gly Leu Thr Met Ile Thr Pro Asp Asn Thr Met Thr Leu Glu Gln
        675                 680                 685

Tyr Val Ala Ala Tyr Asp Pro Ala Thr Met Cys Ser Asn His Trp Val
    690                 695                 700

Gly Ala Ala Lys Met Gly Thr Ser Ser Ser Thr Ala Val Val Asp Glu
705                 710                 715                 720

Asn Ala Lys Val Phe Asn Thr Asp Asn Leu Phe Ile Val Asp Ala Ser
                725                 730                 735

Ile Ile Pro Ser Leu Pro Ile Gly Asn Pro Gln Gly Val Leu Met Ser
            740                 745                 750

Ala Ala Glu Gln Ala Val Ser Arg Ile Leu Ala Leu Ala Gly Gly Pro
        755                 760                 765

<210> 5
<211> 773
<212> PRT
<213> Phanerochaete chrysosporium

<400> 5

Met Leu Gly Arg Ser Leu Leu Ala Leu Leu Pro Phe Val Gly Leu Ala
1               5                   10                  15

Phe Ser Gln Ser Ala Ser Gln Phe Thr Asp Pro Thr Thr Gly Phe Gln
            20                  25                  30

Phe Thr Gly Ile Thr Asp Pro Val His Asp Val Thr Tyr Gly Phe Val
        35                  40                  45

Phe Pro Pro Leu Ala Thr Ser Gly Ala Gln Ser Thr Glu Phe Ile Gly
    50                  55                  60

Glu Val Val Ala Pro Ile Ala Ser Lys Trp Ile Gly Ile Ala Leu Gly
65                  70                  75                  80

Gly Ala Met Asn Asn Asp Leu Leu Leu Val Ala Trp Ala Asn Gly Asn
                85                  90                  95

Gln Ile Val Ser Ser Thr Arg Trp Ala Thr Gly Tyr Val Gln Pro Thr
            100                 105                 110

Ala Tyr Thr Gly Thr Ala Thr Leu Thr Thr Leu Pro Glu Thr Thr Ile
        115                 120                 125

Asn Ser Thr His Trp Lys Trp Val Phe Arg Cys Gln Gly Cys Thr Glu
    130                 135                 140

Trp Asn Asn Gly Gly Gly Ile Asp Val Thr Ser Gln Gly Val Leu Ala
145                 150                 155                 160

Trp Ala Phe Ser Asn Val Ala Val Asp Asp Pro Ser Asp Pro Gln Ser

                165                 170                 175

Thr Phe Ser Glu His Thr Asp Phe Gly Phe Phe Gly Ile Asp Tyr Ser
            180                 185                 190

Thr Ala His Ser Ala Asn Tyr Gln Asn Tyr Leu Asn Gly Asp Ser Gly
        195                 200                 205

Asn Pro Thr Thr Thr Ser Thr Lys Pro Thr Ser Thr Ser Ser Ser Val
    210                 215                 220

Thr Thr Gly Pro Thr Val Ser Ala Thr Pro Tyr Asp Tyr Ile Ile Val
225                 230                 235                 240

Gly Ala Gly Pro Gly Gly Ile Ile Ala Ala Asp Arg Leu Ser Glu Ala
                245                 250                 255

Gly Lys Lys Val Leu Leu Leu Glu Arg Gly Gly Pro Ser Thr Lys Gln
            260                 265                 270

Thr Gly Gly Thr Tyr Val Ala Pro Trp Ala Thr Ser Ser Gly Leu Thr
        275                 280                 285

Lys Phe Asp Ile Pro Gly Leu Phe Glu Ser Leu Phe Thr Asp Ser Asn
    290                 295                 300

Pro Phe Trp Trp Cys Lys Asp Ile Thr Val Phe Ala Gly Cys Leu Val
305                 310                 315                 320

Gly Gly Gly Thr Ser Val Asn Gly Ala Leu Tyr Trp Tyr Pro Asn Asp
                325                 330                 335

Gly Asp Phe Ser Ser Ser Val Gly Trp Pro Ser Ser Trp Thr Asn His
            340                 345                 350

Ala Pro Tyr Thr Ser Lys Leu Ser Ser Arg Leu Pro Ser Thr Asp His
        355                 360                 365

Pro Ser Thr Asp Gly Gln Arg Tyr Leu Glu Gln Ser Phe Asn Val Val
    370                 375                 380

Ser Gln Leu Leu Lys Gly Gln Gly Tyr Asn Gln Ala Thr Ile Asn Asp
385                 390                 395                 400

Asn Pro Asn Tyr Lys Asp His Val Phe Gly Tyr Ser Ala Phe Asp Phe
                405                 410                 415

Leu Asn Gly Lys Arg Ala Gly Pro Val Ala Thr Tyr Leu Gln Thr Ala
            420                 425                 430

Leu Ala Arg Pro Asn Phe Thr Phe Lys Thr Asn Val Met Val Ser Asn

        435                 440                 445

Val Val Arg Asn Gly Ser Gln Ile Leu Gly Val Gln Thr Asn Asp Pro
    450                 455                 460

Thr Leu Gly Pro Asn Gly Phe Ile Pro Val Thr Pro Lys Gly Arg Val
465                 470                 475                 480

Ile Leu Ser Ala Gly Ala Phe Gly Thr Ser Arg Ile Leu Phe Gln Ser
                485                 490                 495

Gly Ile Gly Pro Thr Asp Met Ile Gln Thr Val Gln Ser Asn Pro Thr
            500                 505                 510

Ala Ala Ala Ala Leu Pro Pro Gln Asn Gln Trp Ile Asn Leu Pro Val
        515                 520                 525

Gly Met Asn Ala Gln Asp Asn Pro Ser Ile Asn Leu Val Phe Thr His
    530                 535                 540

Pro Ser Ile Asp Ala Tyr Glu Asn Trp Ala Asp Val Trp Ser Asn Pro
545                 550                 555                 560

Arg Pro Ala Asp Ala Ala Gln Tyr Leu Ala Asn Gln Ser Gly Val Phe
                565                 570                 575

Ala Gly Ala Ser Pro Lys Leu Asn Phe Trp Arg Ala Tyr Ser Gly Ser
            580                 585                 590

Asp Gly Phe Thr Arg Tyr Ala Gln Gly Thr Val Arg Pro Gly Ala Ala
        595                 600                 605

Ser Val Asn Ser Ser Leu Pro Tyr Asn Ala Ser Gln Ile Phe Thr Ile
    610                 615                 620

Thr Val Tyr Leu Ser Thr Gly Ile Gln Ser Arg Gly Arg Ile Gly Ile
625                 630                 635                 640

Asp Ala Ala Leu Arg Gly Thr Val Leu Thr Pro Pro Trp Leu Val Asn
                645                 650                 655

Pro Val Asp Lys Thr Val Leu Leu Gln Ala Leu His Asp Val Val Ser
            660                 665                 670

Asn Ile Gly Ser Ile Pro Gly Leu Thr Met Ile Thr Pro Asp Val Thr
        675                 680                 685

Gln Thr Leu Glu Glu Tyr Val Asp Ala Tyr Asp Pro Ala Thr Met Asn
    690                 695                 700

Ser Asn His Trp Val Ser Ser Thr Thr Ile Gly Ser Ser Pro Gln Ser

705                 710                 715                 720

Ala Val Val Asp Ser Asn Val Lys Val Phe Gly Thr Asn Asn Leu Phe
                725                 730                 735

Ile Val Asp Ala Gly Ile Ile Pro His Leu Pro Thr Gly Asn Pro Gln
            740                 745                 750

Gly Thr Leu Met Ser Ala Ala Glu Gln Ala Ala Ala Lys Ile Leu Ala
        755                 760                 765

Leu Ala Gly Gly Pro
    770

<210>  6
<211>  768
<212>  PRT
<213>  Grifola frondosa

<400>  6

Met Phe Gly His Leu Leu Leu Ala Leu Leu Pro Leu Val Gly Ser Ala
1               5                   10                  15

Phe Ala Gln Ser Gly Ser Ile Tyr Thr Asp Pro Gly Asn Gly Phe Thr
            20                  25                  30

Phe Asp Gly Ile Thr Asp Pro Val Tyr Asp Val Thr Tyr Gly Val Ile
        35                  40                  45

Phe Pro Thr Asp Thr Thr Ser Thr Glu Phe Ile Gly Glu Ile Val Ala
    50                  55                  60

Pro Val Ala Ala Gln Trp Ile Gly Val Ala Leu Gly Gly Ala Met Ile
65                  70                  75                  80

Asp Asn Leu Leu Leu Val Val Trp Thr Asn Gly Asn Thr Ile Val Ser
                85                  90                  95

Ser Thr Arg Tyr Ala Thr Asp Tyr Ile Gln Pro Val Pro Tyr Ala Gly
            100                 105                 110

Pro Thr Leu Thr Thr Leu Pro Ser Ser Ser Val Asn Ser Thr His Trp
        115                 120                 125

Lys Phe Val Phe Arg Cys Gln Asn Cys Thr Ser Trp Leu Gly Gly Gly
    130                 135                 140

Ser Ile Pro Val Ser Gly Ser Gly Val Leu Ala Trp Ala Tyr Ser Ser
145                 150                 155                 160

Ile Pro Val Asp Asp Pro Ala Asp Pro Asn Ser Asp Phe Leu Glu His
                165                 170                 175

Thr Asp Phe Gly Phe Phe Gly Met Asn Phe Ala Asp Ala His Thr Ser
            180                 185                 190

Asn Tyr Asn Asn Tyr Leu Asn Gly Asn Ala Gly Thr Ser Thr Pro Pro
        195                 200                 205

Thr Gly Ser Pro Thr Thr Thr Thr Thr Ser Pro Thr Thr Gly Pro Thr
    210                 215                 220

Thr Pro Ala Thr Pro Tyr Asp Tyr Val Ile Val Gly Ala Gly Pro Gly
225                 230                 235                 240

Gly Ile Ile Ala Ala Asp Arg Leu Ser Glu Ala Gly Lys Lys Val Leu
                245                 250                 255

Leu Leu Glu Arg Gly Gly Pro Ser Thr Gly Glu Thr Gly Gly Thr Tyr
            260                 265                 270

Thr Ala Pro Trp Ala Ala Gly Thr Asn Leu Thr Lys Phe Asp Ile Pro
        275                 280                 285

Gly Leu Phe Glu Ser Met Phe Thr Asp Ser Asn Pro Trp Tyr Trp Cys
    290                 295                 300

Lys Asp Ile Asn Phe Phe Ala Gly Cys Leu Leu Gly Gly Gly Thr Ser
305                 310                 315                 320

Val Asn Gly Ala Leu Tyr Trp Tyr Pro Thr Asp Ser Asp Phe Ser Thr
                325                 330                 335

Asn Asn Gly Trp Pro Asn Ser Trp Gly Tyr His Gln Pro Tyr Thr Ser
            340                 345                 350

Ala Met Gln Ala Arg Leu Pro Ser Thr Asp His Pro Ser Thr Asp Gly
        355                 360                 365

Gln Leu Tyr Leu Glu Gln Ser Ala Asn Val Val Met Gln Leu Leu Asn
    370                 375                 380

Gly Gln Gly Tyr Arg Gln Val Thr Ile Asn Asn Asp Val Asn Ser Lys
385                 390                 395                 400

Asp His Val Tyr Gly Tyr Ser Ala Phe Asp Phe Leu Asn Gly Lys Arg
                405                 410                 415

Gly Gly Pro Val Ala Thr Tyr Leu Gln Thr Ala Asn Ala Arg Ser Asn
            420                 425                 430

Phe Val Tyr Lys Asp Tyr Thr Met Val Ser Asn Ile Val Arg Asn Gly
        435                 440                 445

Ser Gln Ile Thr Gly Val Lys Thr Asn Asp Thr Ser Leu Gly Pro Asn
    450                 455                 460

Gly Val Val Pro Leu Thr Pro Asn Gly Arg Val Ile Leu Ser Ala Gly
465                 470                 475                 480

Ser Phe Gly Ser Pro Arg Ile Leu Phe Gln Ser Gly Ile Gly Pro Thr
                485                 490                 495

Asp Met Leu Gln Thr Val Gln Gly Asn Pro Ala Ala Ala Pro Asn Leu
            500                 505                 510

Pro Pro Gln Ser Glu Trp Ile Asn Leu Pro Val Gly Met Ala Val Ser
        515                 520                 525

Asp Asn Pro Ser Ile Asn Leu Val Phe Thr His Pro Ser Ile Asp Ala
    530                 535                 540

Tyr Asp Asn Trp Ala Asp Val Trp Ser Asp Pro Arg Pro Ala Asp Ala
545                 550                 555                 560

Ala Gln Tyr Leu Ala Ser Gln Ser Gly Val Phe Ala Gly Ala Ser Pro
                565                 570                 575

Lys Leu Asn Phe Trp Arg Ala Tyr Ser Gly Ile Asp Ala Asn Gln Arg
            580                 585                 590

Trp Ala Gln Gly Thr Val Arg Pro Gly Ala Ala Ser Val Asn Thr Thr
        595                 600                 605

Leu Pro Tyr Asn Ala Ser Glu Ile Phe Thr Ile Thr Val Tyr Leu Ser
    610                 615                 620

Gln Gly Ile Thr Ser Arg Gly Arg Ile Gly Ile Asn Ala Gly Leu Glu
625                 630                 635                 640

Ala Thr Ala Leu Val Asn Pro Trp Leu Thr Asp Pro Val Asp Lys Thr
                645                 650                 655

Val Leu Ile Gln Ala Leu Asn Asp Val Val Ser Asn Met Asn Ser Ile
            660                 665                 670

Ser Gly Leu Thr Met Ile Thr Pro Asp Ser Gly Met Thr Ile Glu Glu
        675                 680                 685

Tyr Val Asp Leu Tyr Asp Pro Gly Thr Met Cys Ser Asn His Trp Val
    690                 695                 700

Gly Ser Asn Lys Met Gly Thr Asn Ser Ser Thr Ala Val Val Asp Glu
705                 710                 715                 720

Asn Ala Lys Val Phe Asn Thr Asn Asn Leu Phe Val Val Asp Ala Ser
                725                 730                 735

Ile Ile Pro Ala Leu Pro Val Gly Asn Pro His Gly Met Leu Met Ser
            740                 745                 750

Ala Ala Glu Gln Ala Val Ala Asn Ile Leu Ala Leu Ala Gly Gly Pro
        755                 760                 765

<210>  7
<211>  777
<212>  PRT
<213>  Irpex lacteus

<400>  7

Met Leu Arg Arg Thr Leu Leu Ala Leu Leu Pro Leu Ile Gly Thr Ala
1               5                   10                  15

Leu Ser Gln Ser Ala Ser Asn Tyr Ile Asp Pro Asp Asn Gly Phe Gln
            20                  25                  30

Phe Thr Gly Val Thr Asp Ala Glu Thr Gln Val Thr Tyr Gly Val Thr
        35                  40                  45

Phe Pro Pro Leu Ala Thr Ser Gly Ala Gln Ser Thr Glu Phe Ile Gly
    50                  55                  60

Glu Val Val Ala Pro Val Ala Ala Lys Trp Val Gly Ile Ala Leu Ala
65                  70                  75                  80

Gly Ala Met Leu Gln Asp Leu Leu Leu Val Ala Trp Pro Asn Ala Gly
                85                  90                  95

Lys Ile Val Ser Ser Thr Arg Ile Ala Ser Asp Tyr Val Gln Pro Thr
            100                 105                 110

Ala Tyr Thr Gly Ala Ala Thr Leu Thr Thr Leu Pro Glu Thr Thr Val
        115                 120                 125

Asn Ala Thr His Trp Lys Trp Val Phe Arg Cys Gln Gly Cys Thr Ser
    130                 135                 140

Trp Thr Ser Pro Ser Gly Ser Thr Gly Ser Ile Ser Val Asp Gly Ser
145                 150                 155                 160

Gly Val Leu Ala Trp Ala Tyr Ser Ser Val Gly Val Asp Asp Pro Thr
                165                 170                 175

Asp Pro Glu Ser Thr Phe Gln Glu His Thr Ser Phe Gly Phe Phe Gly
            180                 185                 190

Ile Asp Tyr Ser Gln Ala His Thr Ser Asn Tyr Gln Asn Tyr Leu Asp
        195                 200                 205

Gly Asn Pro Gly Thr Pro Thr Ser Ala Pro Ser Gly Pro Ser Ser Thr
    210                 215                 220

Thr Thr Thr Val Pro Thr Gly Pro Thr Thr Pro Ala Val Ala Tyr Asp
225                 230                 235                 240

Tyr Ile Val Val Gly Ala Gly Pro Gly Gly Ile Ile Ala Ala Asp Arg
                245                 250                 255

Leu Ser Glu Ala Gly Lys Lys Val Leu Leu Leu Glu Arg Gly Gly Pro
            260                 265                 270

Ser Thr Lys Val Thr Gly Gly Thr Tyr Val Pro Ser Trp Ala Gly Ser
        275                 280                 285

Thr Gly Leu Thr Lys Phe Asp Ile Pro Gly Val Phe Glu Ser Met Phe
    290                 295                 300

Thr Asp Ser Asn Pro Phe Trp Trp Cys Lys Asp Val Thr Val Phe Ala
305                 310                 315                 320

Gly Cys Leu Ile Gly Gly Gly Thr Ser Ile Asn Gly Ala Leu Tyr Trp
                325                 330                 335

Tyr Pro Asn Ser Asp Asp Phe Ser Thr Ala Ala Gly Trp Pro Ser Ser
            340                 345                 350

Trp Thr Asn His Gly Pro Tyr Thr Ala Lys Leu Lys Ala Arg Leu Pro
        355                 360                 365

Ser Thr Asp His Pro Ser Ala Asp Gly Lys Arg Tyr Leu Glu Gln Thr
    370                 375                 380

Ala Asp Leu Val Gly Gln Leu Leu Lys Gly Gln Gly Tyr Ser Asn Ile
385                 390                 395                 400

Thr Ile Asn Asp Asn Pro Asp Tyr Lys Asp His Val Tyr Gly Tyr Ala
                405                 410                 415

Ala Tyr Asp Phe Leu Gly Gly Leu Arg Gly Gly Pro Val Ala Thr Tyr
            420                 425                 430

Leu Gln Thr Ala Leu Ala Arg Pro Asn Phe Thr Leu Lys Gln Tyr Thr
        435                 440                 445

Leu Val Thr Asn Val Val Arg Asn Gly Ser Thr Ile Thr Gly Val Gln
    450                 455                 460

Thr Asn Asp Thr Ser Leu Gly Pro Asn Gly Ile Ile Pro Leu Thr Lys
465                 470                 475                 480

Asn Gly Arg Val Ile Leu Ala Ala Gly Ser Leu Gln Thr Pro Arg Leu
                485                 490                 495

Leu Phe Gln Ser Gly Ile Gly Pro Thr Asp Gln Ile Asn Leu Val Lys
            500                 505                 510

Gly Asn Thr Asp Ala Ala Ser Arg Leu Pro Pro Gln Ser Asp Trp Ile
        515                 520                 525

Asn Leu Pro Val Gly Tyr Asn Ala Gln Asp Asn Pro Ser Ile Asn Leu
    530                 535                 540

Val Phe Thr His Pro Ser Ile Asp Ala Tyr Asp Asn Trp Ala Asp Val
545                 550                 555                 560

Trp Thr Asn Pro Pro Ser Ala Asp Val Ala Gln Tyr Leu Gly Ser Arg
                565                 570                 575

Ser Gly Ile Leu Ala Ser Ala Ser Pro Arg Leu Asn Phe Trp Arg Ala
            580                 585                 590

Tyr Gly Gly Ser Asp Gly Val Val Arg Tyr Ala Gln Gly Thr Val Arg
        595                 600                 605

Pro Gly Ala Ala Ser Val Asn Thr Thr Leu Pro Tyr Asn Ala Ser Asn
    610                 615                 620

Ile Phe Thr Ile Thr Val Tyr Leu Ser Thr Gly Ile Gln Ser Arg Gly
625                 630                 635                 640

Arg Val Gly Ile Asp Ala Ser Leu Arg Ala Lys Pro Leu Val Asn Pro
                645                 650                 655

Trp Leu Val Asp Pro Val Asp Lys Gln Val Leu Leu Gln Ala Leu Asn
            660                 665                 670

Asp Val Val Ser Asn Ile Lys Ser Ile Gln Asn Leu Thr Leu Ile Thr
        675                 680                 685

Pro Asp Val His Gln Thr Ile Glu Glu Tyr Val Asp Ala Tyr Asp Pro
    690                 695                 700

Ala Thr Met Asn Ser Asn His Trp Val Ser Thr Ala Thr Ile Gly Thr
705                 710                 715                 720

Ser Ala Ser Asn Ala Val Val Asp Gln Asn Thr Lys Val Phe Asn Thr
                725                 730                 735

Asn Asn Leu Phe Ile Val Asp Ala Ser Ile Ile Pro His Leu Pro Val
            740                 745                 750

Gly Asn Pro His Gly Ala Leu Met Ser Ala Ala Glu Gln Ala Ala Ala
        755                 760                 765

Lys Ile Leu Ala Leu Ala Gly Gly Pro
    770                 775

<210> 8
<211> 269
<212> PRT
<213> Thermomyces lanuginosus

<400> 8

Glu Val Ser Gln Asp Leu Phe Asn Gln Phe Asn Leu Phe Ala Gln Tyr
1               5                   10                  15

Ser Ala Ala Ala Tyr Cys Gly Lys Asn Asn Asp Ala Pro Ala Gly Thr
            20                  25                  30

Asn Ile Thr Cys Thr Gly Asn Ala Cys Pro Glu Val Glu Lys Ala Asp
        35                  40                  45

Ala Thr Phe Leu Tyr Ser Phe Glu Asp Ser Gly Val Gly Asp Val Thr
    50                  55                  60

Gly Phe Leu Ala Leu Asp Asn Thr Asn Lys Leu Ile Val Leu Ser Phe
65                  70                  75                  80

Arg Gly Ser Arg Ser Ile Glu Asn Trp Ile Gly Asn Leu Asn Phe Asp
                85                  90                  95

Leu Lys Glu Ile Asn Asp Ile Cys Ser Gly Cys Arg Gly His Asp Gly
            100                 105                 110

Phe Thr Ser Ser Trp Arg Ser Val Ala Asp Thr Leu Arg Gln Lys Val
        115                 120                 125

Glu Asp Ala Val Arg Glu His Pro Asp Tyr Arg Val Val Phe Thr Gly
    130                 135                 140

His Ser Leu Gly Gly Ala Leu Ala Thr Val Ala Gly Ala Asp Leu Arg
145                 150                 155                 160

Gly Asn Gly Tyr Asp Ile Asp Val Phe Ser Tyr Gly Ala Pro Arg Val
                165                 170                 175

Gly Asn Arg Ala Phe Ala Glu Phe Leu Thr Val Gln Thr Gly Gly Thr
            180                 185                 190

Leu Tyr Arg Ile Thr His Thr Asn Asp Ile Val Pro Arg Leu Pro Pro
        195                 200                 205

Arg Glu Phe Gly Tyr Ser His Ser Ser Pro Glu Tyr Trp Ile Lys Ser
    210                 215                 220

Gly Thr Leu Val Pro Val Arg Arg Arg Asp Ile Val Lys Ile Glu Gly
225                 230                 235                 240

Ile Asp Ala Thr Gly Gly Asn Asn Gln Pro Asn Ile Pro Asp Ile Pro
                245                 250                 255

Ala His Leu Trp Tyr Phe Gly Leu Ile Gly Thr Cys Leu
            260                 265

<210>  9
<211>  484
<212>  PRT
<213>  Bacillus strain NCIB 12513

<400>  9

His His Asn Gly Thr Asn Gly Thr Met Met Gln Tyr Phe Glu Trp His
1               5                   10                  15

Leu Pro Asn Asp Gly Asn His Trp Asn Arg Leu Arg Asp Asp Ala Ser
            20                  25                  30

Asn Leu Arg Asn Arg Gly Ile Thr Ala Ile Trp Ile Pro Pro Ala Trp
        35                  40                  45

Lys Gly Thr Ser Gln Asn Asp Val Gly Tyr Gly Ala Tyr Asp Leu Tyr
    50                  55                  60

Asp Leu Gly Glu Phe Asn Gln Lys Gly Thr Val Arg Thr Lys Tyr Gly
65                  70                  75                  80

Thr Arg Ser Gln Leu Glu Ser Ala Ile His Ala Leu Lys Asn Asn Gly
                85                  90                  95

Val Gln Val Tyr Gly Asp Val Val Met Asn His Lys Gly Gly Ala Asp
            100                 105                 110

Ala Thr Glu Asn Val Leu Ala Val Glu Val Asn Pro Asn Asn Arg Asn
        115                 120                 125

Gln Glu Ile Ser Gly Asp Tyr Thr Ile Glu Ala Trp Thr Lys Phe Asp
    130                 135                 140

Phe Pro Gly Arg Gly Asn Thr Tyr Ser Asp Phe Lys Trp Arg Trp Tyr
145                 150                 155                 160

His Phe Asp Gly Val Asp Trp Asp Gln Ser Arg Gln Phe Gln Asn Arg

                165                 170                 175

Ile Tyr Lys Phe Arg Gly Asp Gly Lys Ala Trp Asp Trp Glu Val Asp
            180                 185                 190

Ser Glu Asn Gly Asn Tyr Asp Tyr Leu Met Tyr Ala Asp Val Asp Met
        195                 200                 205

Asp His Pro Glu Val Val Asn Glu Leu Arg Arg Trp Gly Glu Trp Tyr
    210                 215                 220

Thr Asn Thr Leu Asn Leu Asp Gly Phe Arg Ile Asp Ala Val Lys His
225                 230                 235                 240

Ile Lys Tyr Ser Phe Thr Arg Asp Trp Leu Thr His Val Arg Asn Ala
                245                 250                 255

Thr Gly Lys Glu Met Phe Ala Val Ala Glu Phe Trp Lys Asn Asp Leu
            260                 265                 270

Gly Ala Leu Glu Asn Tyr Leu Asn Lys Thr Asn Trp Asn His Ser Val
        275                 280                 285

Phe Asp Val Pro Leu His Tyr Asn Leu Tyr Asn Ala Ser Asn Ser Gly
    290                 295                 300

Gly Asn Tyr Asp Met Ala Lys Leu Leu Asn Gly Thr Val Val Gln Lys
305                 310                 315                 320

His Pro Met His Ala Val Thr Phe Val Asp Asn His Asp Ser Gln Pro
                325                 330                 335

Gly Glu Ser Leu Glu Ser Phe Val Gln Glu Trp Phe Lys Pro Leu Ala
            340                 345                 350

Tyr Ala Leu Ile Leu Thr Arg Glu Gln Gly Tyr Pro Ser Val Phe Tyr
        355                 360                 365

Gly Asp Tyr Tyr Gly Ile Pro Thr His Ser Val Pro Ala Met Lys Ala
    370                 375                 380

Lys Ile Asp Pro Ile Leu Glu Ala Arg Gln Asn Phe Ala Tyr Gly Thr
385                 390                 395                 400

Gln His Asp Tyr Phe Asp His His Asn Ile Ile Gly Trp Thr Arg Glu
                405                 410                 415

Gly Asn Thr Thr His Pro Asn Ser Gly Leu Ala Thr Ile Met Ser Asp
            420                 425                 430

Gly Pro Gly Gly Glu Lys Trp Met Tyr Val Gly Gln Asn Lys Ala Gly

435                 440                 445

Gln Val Trp His Asp Ile Thr Gly Asn Lys Pro Gly Thr Val Thr Ile
    450                 455                 460

Asn Ala Asp Gly Trp Ala Asn Phe Ser Val Asn Gly Gly Ser Val Ser
465                 470                 475                 480

Ile Trp Val Lys

<210>  10
<211>  773
<212>  PRT
<213>  Bacillus sp AA439

<400>  10

Ala Glu Gly Asn Thr Arg Glu Asp Asn Phe Lys His Leu Leu Gly Asn
1               5                   10                  15

Asp Asn Val Lys Arg Pro Ser Glu Ala Gly Ala Leu Gln Leu Gln Glu
            20                  25                  30

Val Asp Gly Gln Met Thr Leu Val Asp Gln His Gly Glu Lys Ile Gln
        35                  40                  45

Leu Arg Gly Met Ser Thr His Gly Leu Gln Trp Phe Pro Glu Ile Leu
    50                  55                  60

Asn Asp Asn Ala Tyr Lys Ala Leu Ala Asn Asp Trp Glu Ser Asn Met
65                  70                  75                  80

Ile Arg Leu Ala Met Tyr Val Gly Glu Asn Gly Tyr Ala Ser Asn Pro
                85                  90                  95

Glu Leu Ile Lys Ser Arg Val Ile Lys Gly Ile Asp Leu Ala Ile Glu
            100                 105                 110

Asn Asp Met Tyr Val Ile Val Asp Trp His Val His Ala Pro Gly Asp
        115                 120                 125

Pro Arg Asp Pro Val Tyr Ala Gly Ala Glu Asp Phe Phe Arg Asp Ile
    130                 135                 140

Ala Ala Leu Tyr Pro Asn Asn Pro His Ile Ile Tyr Glu Leu Ala Asn
145                 150                 155                 160

Glu Pro Ser Ser Asn Asn Asn Gly Gly Ala Gly Ile Pro Asn Asn Glu
                165                 170                 175

Glu Gly Trp Asn Ala Val Lys Glu Tyr Ala Asp Pro Ile Val Glu Met
            180                 185                 190

Leu Arg Asp Ser Gly Asn Ala Asp Asp Asn Ile Ile Ile Val Gly Ser
        195                 200                 205

Pro Asn Trp Ser Gln Arg Pro Asp Leu Ala Ala Asp Asn Pro Ile Asn
    210                 215                 220

Asp His His Thr Met Tyr Thr Val His Phe Tyr Thr Gly Ser His Ala
225                 230                 235                 240

Ala Ser Thr Glu Ser Tyr Pro Pro Glu Thr Pro Asn Ser Glu Arg Gly
                245                 250                 255

Asn Val Met Ser Asn Thr Arg Tyr Ala Leu Glu Asn Gly Val Ala Val
            260                 265                 270

Phe Ala Thr Glu Trp Gly Thr Ser Gln Ala Asn Gly Asp Gly Gly Pro
        275                 280                 285

Tyr Phe Asp Glu Ala Asp Val Trp Ile Glu Phe Leu Asn Glu Asn Asn
    290                 295                 300

Ile Ser Trp Ala Asn Trp Ser Leu Thr Asn Lys Asn Glu Val Ser Gly
305                 310                 315                 320

Ala Phe Thr Pro Phe Glu Leu Gly Lys Ser Asn Ala Thr Asn Leu Asp
                325                 330                 335

Pro Gly Pro Asp His Val Trp Ala Pro Glu Glu Leu Ser Leu Ser Gly
            340                 345                 350

Glu Tyr Val Arg Ala Arg Ile Lys Gly Val Asn Tyr Glu Pro Ile Asp
        355                 360                 365

Arg Thr Lys Tyr Thr Lys Val Leu Trp Asp Phe Asn Asp Gly Thr Lys
    370                 375                 380

Gln Gly Phe Gly Val Asn Ser Asp Ser Pro Asn Lys Glu Leu Ile Ala
385                 390                 395                 400

Val Asp Asn Glu Asn Asn Thr Leu Lys Val Ser Gly Leu Asp Val Ser
                405                 410                 415

Asn Asp Val Ser Asp Gly Asn Phe Trp Ala Asn Ala Arg Leu Ser Ala
            420                 425                 430

Asp Gly Trp Gly Lys Ser Val Asp Ile Leu Gly Ala Glu Lys Leu Thr
        435                 440                 445

Met Asp Val Ile Val Asp Glu Pro Thr Thr Val Ala Ile Ala Ala Ile
    450                 455                 460

Pro Gln Ser Ser Lys Ser Gly Trp Ala Asn Pro Glu Arg Ala Val Arg
465                 470                 475                 480

Val Asn Ala Glu Asp Phe Val Gln Gln Thr Asp Gly Lys Tyr Lys Ala
                485                 490                 495

Gly Leu Thr Ile Thr Gly Glu Asp Ala Pro Asn Leu Lys Asn Ile Ala
            500                 505                 510

Phe His Glu Glu Asp Asn Asn Met Asn Asn Ile Ile Leu Phe Val Gly
        515                 520                 525

Thr Asp Ala Ala Asp Val Ile Tyr Leu Asp Asn Ile Lys Val Ile Gly
    530                 535                 540

Thr Glu Val Glu Ile Pro Val Val His Asp Pro Lys Gly Glu Ala Val
545                 550                 555                 560

Leu Pro Ser Val Phe Glu Asp Gly Thr Arg Gln Gly Trp Asp Trp Ala
                565                 570                 575

Gly Glu Ser Gly Val Lys Thr Ala Leu Thr Ile Glu Glu Ala Asn Gly
            580                 585                 590

Ser Asn Ala Leu Ser Trp Glu Phe Gly Tyr Pro Glu Val Lys Pro Ser
        595                 600                 605

Asp Asn Trp Ala Thr Ala Pro Arg Leu Asp Phe Trp Lys Ser Asp Leu
    610                 615                 620

Val Arg Gly Glu Asn Asp Tyr Val Ala Phe Asp Phe Tyr Leu Asp Pro
625                 630                 635                 640

Val Arg Ala Thr Glu Gly Ala Met Asn Ile Asn Leu Val Phe Gln Pro
                645                 650                 655

Pro Thr Asn Gly Tyr Trp Val Gln Ala Pro Lys Thr Tyr Thr Ile Asn
            660                 665                 670

Phe Asp Glu Leu Glu Glu Ala Asn Gln Val Asn Gly Leu Tyr His Tyr
        675                 680                 685

Glu Val Lys Ile Asn Val Arg Asp Ile Thr Asn Ile Gln Asp Asp Thr
    690                 695                 700

Leu Leu Arg Asn Met Met Ile Ile Phe Ala Asp Val Glu Ser Asp Phe
705                 710                 715                 720

Ala Gly Arg Val Phe Val Asp Asn Val Arg Phe Glu Gly Ala Ala Thr
                725                 730                 735

Thr Glu Pro Val Glu Pro Glu Pro Val Asp Pro Gly Glu Glu Thr Pro
            740                 745                 750

Pro Val Asp Glu Lys Glu Ala Lys Lys Glu Gln Lys Glu Ala Glu Lys
        755                 760                 765

Glu Glu Lys Glu Glu
    770